# EUROPÄISCHE PATENTSCHRIFT

(11) **EP 1 758 905 B1**
(45) Veröffentlichungstag und Bekanntmachung des Hinweises auf die Patenterteilung: **29.04.2009**
(21) Anmeldenummer: 05707354.6
(22) Anmeldetag: 12.02.2005
(51) Int. Cl.: C07D 473/04, A61K 31/437, A61P 3/10

(54) **8-[3-AMINO-PIPERIDIN-1-YL]-XANTHINE, DEREN HERSTELLUNG UND DEREN VERWENDUNG ALS DPP-IV HEMMER**
8-[3-AMINO-PIPERIDIN-1-YL]-XANTHINE, THE PRODUCTION THEREOF AND THE USE IN THE FORM OF A DDP-IV INHIBITOR
8-[3-AMINO-PIPERIDINE-1-YL]-XANTHINES, LEUR FABRICATION ET LEUR UTILISATION COMME INHIBITEUR DE DPP-IV

(30) Priorität: 18.02.2004 DE 102004008112; 17.03.2004 DE 102004012921; 03.07.2004 DE 102004032263
(43) Veröffentlichungstag der Anmeldung: 07.03.2007
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: HIMMELSBACH, Frank, 88441 Mittelbeberach (DE); LANGKOPF, Elke, 88447 Warthausen (DE); ECKHARDT, Matthias, 88400 Biberach (DE); TADAYYON, Mohammad, SG14 1HQ Hertford (GB); THOMAS, Leo, 88400 Biberach (DE)
(74) Vertreter: Hammann, Heinz
(86) Internationale Anmeldenummer: PCT/EP2005/001427
(87) Internationale Veröffentlichungsnummer: WO 2005/085246

(56) Entgegenhaltungen:
- WO-A-02/068420
- WO-A-20/04018468

## Beschreibung

Gegenstand der vorliegenden Erfindung sind neue substituierte Xanthine der allgemeinen Formel 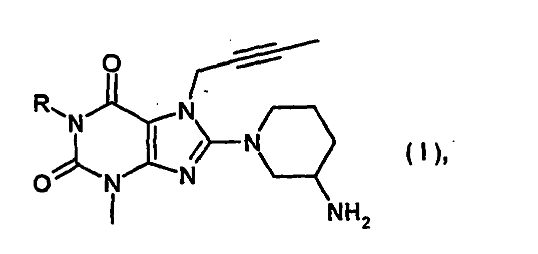 deren Tautomere, Enantiomere, Diastereomere, deren Gemische und deren Salze, insbesonders deren physiologisch verträgliche Salze mit anorganischen oder organischen Säuren, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

Xanthinderivate mit einer hemmenden Wirkung auf DPP-IV sind bereits aus WO 02/068420, WO 02/02560, WO 03/004496, WO 03/024965, WO 04/018468, WO 04/048379, JP 2003300977 und EP 1 338 595 bekannt.

In der obigen Formel I bedeutet
R eine Benzyl-, 2-Fluorbenzyl-, 3-Fluorbenzyl-, 4-Fluorbenzyl-, 2,6-Difluor-benzyl-, 3,4-Difluor-benzyl-, 2-Chlorbenzyl-, 3-Chlorbenzyl- oder 4-Chlorbenzyl-Gruppe,
eine 2-Trifluormethyl-benzyl-, 3-Trifluormethyl-benzyl- oder 4-Trifluormethyl-benzyl-Gruppe,
eine 3-Trifluormethoxy-benzyl- oder 4-Trifluormethoxy-benzyl-Gruppe,
eine 2-Cyanobenzyl-, 3-Cyanobenzyl- oder 4-Cyanobenzyl-Gruppe,
eine 2,6-Dicyanobenzyl-, 3,4-Dicyanobenzyl-, 3,5-Dicyanobenzyl-, 2-Trifluormethyl-4-cyano-benzyl-, 3-Nitro-4-cyano-benzyl-, 2-Cyano-3-methoxy-benzyl-, 2-Cyano-4-methoxy-benzyl-, 2-Cyano-5-methoxy-benzyl-, 2-Cyano-4-fluor-benzyl-, 2-Cyano-5-fluor-benzyl-, 2-Cyano-6-fluor-benzyl-, 3-Cyano-4-fluor-benzyl-, 4-Cyano-3-fluorbenzyl-, 2-Fluor-4-cyano-benzyl-, 2-Cyano-3-chlorbenzyl-, 2-Chlor-4-cyano-benzyl- oder 2-Cyano-4-brombenzyl-Gruppe,
eine 2-Methoxy-benzyl-, 3-Methoxy-benzyl-, 4-Methoxy-benzyl-, 2-Fluor-3-methoxy-benzyl-, 2-Fluor-4-methoxy-benzyl-, 2-Fluor-5-methoxy-benzyl-, 3-Fluor-4-methoxy-benzyl-, 3,4-Dimethoxy-benzyl-, 3,5-Dimethoxybenzyl- oder 3,4-Dimethoxy-6-fluorbenzyl-Gruppe,
eine (Benzo[1,3]dioxol-5-yl)methyl-Gruppe,
eine [(4-Cyano-benzo[1,3]dioxol-5-yl)methyl-Gruppe,
eine 2-(3-Cyclopropyloxy-phenyl)-2-oxo-ethyl-; 2-(3-Cyclopropylmethoxy-phenyl)-2-oxo-ethyl- oder 2-(3-Cyclobutyloxy-phenyl)-2-oxo-ethyl-Gruppe,
eine 2-Oxo-2-[2-(pyridin-3-yl)-phenyl]-ethyl- oder 2-Oxo-2-[2-(pyridin-4-yl)-phenyl]-ethyl-Gruppe,
eine (3-Cyano-naphthalin-1-yl)methyl-, (1,4-Dicyano-naphthalin-2-yl)methyl- oder (2,4-Dimethoxy-naphthalin-1-yl)methyl-Gruppe,
eine (Furan-2-yl)methyl-, (Furan-3-yl)methyl-, (5-Brom-furan-2-yl)methyl-, (5-Methyl-furan-2-yl)methyl-, (5-Cyano-furan-2-yl)methyl- oder (5-Methoxycarbonyl-furan-2-yl)methyl-Gruppe,
eine (Pyridin-2-yl)methyl-, (6-Fluor-pyridin-2-yl)methyl- oder (5-Methoxy-pyridin-2-yl)methyl-Gruppe,
eine (3-Cyanopyridin-2-yl)methyl-, (6-Cyanopyridin-2-yl)methyl-, (5-Cyano-pyridin-2-yl)methyl-, (4-Cyano-pyridin-2-yl)methyl-, (4-Cyano-pyridin-3-yl)methyl-, (3-Cyano-pyridin-4-yl)methyl-, (2-Cyano-pyridin-3-yl)methyl-, (2-Cyano-pyridin-4-yl)methyl-, (5-Cyano-pyridin-3-yl)methyl-, (6-Cyano-pyridin-3-yl)methyl- oder (5-Cyano-6-methoxy-pyridin-2-yl)methyl-Gruppe,
eine (6-Phenyl-pyridin-2-yl)methyl- oder eine ([2,2']Bipyridinyl-6-yl)methyl-Gruppe,
eine (Pyrimidin-2-yl)methyl-, (4-Methyl-pyrimidin-2-yl)methyl- oder (4,6-Dimethyl-pyrimidin-2-yl)methyl-Gruppe,
eine (2-Phenyl-pyrimidin-4-yl)methyl- oder (4-Phenyl-pyrimidin-2-yl)methyl-Gruppe,
eine [(1-Methyl-1 H-benzotriazol-5-yl)methyl]-Gruppe,
eine (6-Fluor-chinolin-2-yl)methyl-, (7-Fluor-chinolin-2-yl)methyl-, (2-Methyl-chinolin-4-yl)methyl-, (3-Cyano-chinolin-2-yl)methyl-, (3-Cyano-4-methyl-chinolin-2-yl)methyl-, (4-Cyano-chinotin-2-yl)methyl-, (5-Cyano-chinolin-2-yl)methyl-, (8-Cyano-chinolin-2-yl)methyl-, (6-Amino-chinolin-2-yl)methyl-, (8-Amino-chinolin-2-yl)methyl-, (4-Methoxy-chinolin-2-yl)methyl-, (6-Methoxy-chinolin-2-yl)methyl-, (6,7-Dimethoxy-chinolin-2-yl)methyl- oder (8-Cyano-chinolin-7-yl)methyl-Gruppe, eine (1-Cyano-isochinolin-3-yl)methyl-, (4-Cyano-isochinolin-1-yl)methyl- (4-Cyano-isochinolin-3-yl)methyl- oder [(4-(Pyridin-2-yl)-isochinolin-1-yl]methyl-Gruppe,
eine (Chinazolin-6-yl)methyl-, (Chinazolin-7-yl)methyl-, (2-Methyl-chinazolin-4-yl)methyl-, (4,5-Dimethyl-chinazolin-2-yl)methyl-, (4-Ethyl-chinazolin-2-yl)methyl-, (4-Cyclopropyl-chinazolin-2-yl)methyl-, (2-Phenyl-chinazolin-4-yl)methyl-, (4-Cyano-chinazolin-2-yl)methyl-, (4-Phenylamino-chinazolin-2-yl)methyl- oder (4-Benzylamino-chinazolin-2-yl)methyl-Gruppe,
eine (Chinoxalin-5-yl)methyl- (Chinoxalin-6-yl)methyl- oder (2,3-Dimethyl-chinoxalin-6-yl)methyl-Gruppe, oder
eine ([1,5]Naphthyridin-3-yl)methyl-Gruppe,
deren Tautomere, Enantiomere, Diastereomere, deren Gemische und deren Salze.

Bevorzugt sind Verbindungen der allgemeinen Formel 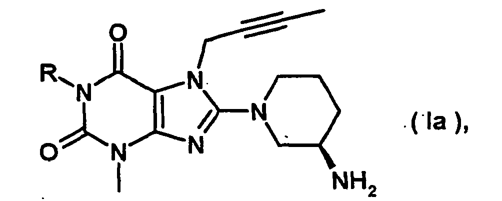 in der R wie oben erwähnt definiert ist, sowie deren Tautomere und Salze.

Ebenfalls bevorzugt sind Verbindungen der allgemeinen Formel 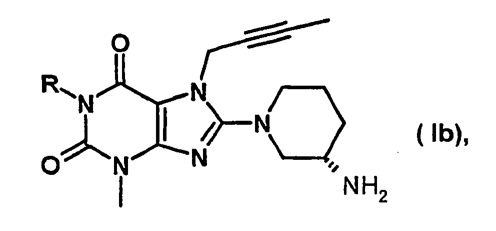 in der R wie oben erwähnt definiert ist, sowie deren Tautomere und Salze.

Erfindungsgemäß erhält man die Verbindungen der allgemeinen Formel I nach an sich bekannten Verfahren, beispielsweise nach folgenden Verfahren:
a) Umsetzung einer Verbindung der allgemeinen Formel 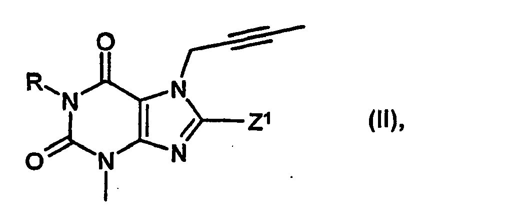 in der
   R wie eingangs erwähnt definiert ist und
   Z¹ eine Austrittsgruppe wie ein Halogenatom, eine substituierte Hydroxy-, Mercapto-, Sulfinyl-, Sulfonyl- oder Sulfonyloxygruppe wie ein Chlor- oder Bromatom, eine Methansulfonyl- oder Methansulfonyloxygruppe darstellt, mit 3-Aminopiperidin, dessen Enantiomeren oder dessen Salzen.
   Die Umsetzung wird zweckmäßigerweise in einem Lösungsmittel wie Isopropanol, Butanol, Tetrahydrofuran, Dioxan, Dimethylformamid, Dimethylsulfoxid, Ethylenglycolmonomethylether, Ethylenglycoldiethylether oder Sulfolan gegebenenfalls in Gegenwart einer anorganischen oder tertiären organischen Base, z.B. Natriumcarbonat, Kaliumcarbonat oder Kaliumhydroxid, einer tertiären organischen Base, z.B. Triethylamin, oder in Gegenwart von N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid oder einem Katalysator auf Palladiumbasis bei Temperaturen zwischen -20 und 180°C, vorzugsweise jedoch bei Temperaturen zwischen -10 und 120°C, durchgeführt. Die Umsetzung kann jedoch auch ohne Lösungsmittel oder in einem Überschuß des 3-Aminopiperidins durchgeführt werden.
b) Entschützung einer Verbindung der allgemeinen Formel 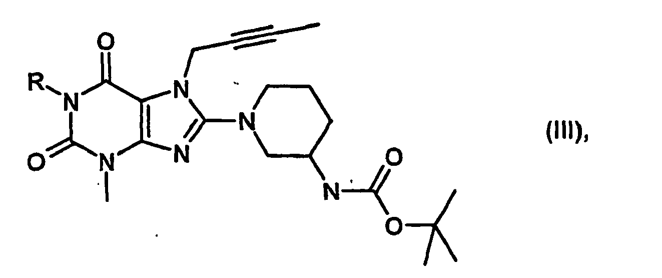 in der R wie eingangs definiert ist.

Die Abspaltung des tert.-Butyloxycarbonylrestes erfolgt vorzugsweise durch Behandlung mit einer Säure wie Trifluoressigsäure oder Salzsäure oder durch Behandlung mit Bromtrimethylsilan oder lodtrimethylsilan gegebenenfalls unter Verwendung eines Lösungsmittels wie Methylenchlorid, Essigester, Dioxan, Methanol, Isopropanol oder Diethylether bei Temperaturen zwischen 0 und 80°C.

Bei den vorstehend beschriebenen Umsetzungen können gegebenenfalls vorhandene reaktive Gruppen wie Amino-, Alkylamino- oder Iminogruppen während der Umsetzung durch übliche Schutzgruppen geschützt werden, welche nach der Umsetzung wieder abgespalten werden.

Beispielsweise kommen als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin, Ethanolamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So-lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II und

III sind entweder literaturbekannt oder man erhält diese nach an sich literaturbekannten Verfahren (siehe Beispiele I bis XXV).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:

Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2" , erschienen in Proc. Natl. Acad. Sci. Vol. 90, Seiten 5757-5761 (1993), durchgeführt. Der Zellextrakt wurde von in einem Puffer (10 mM Tris HCI, 0.15 M NaCl, 0.04 t.i.u. Aprotinin, 0.5% Nonidet-P40, pH 8.0) solubilisierten Zellen durch Zentrifugation bei 35,000 g für 30 Minuten bei 4°C (zur Entfernung von Zelltrümmern) gewonnen.

Der DPP-IV Assay wurde wie folgt durchgeführt:
50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCI pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung | DPP IV-Hemmung |
|---|---|
| (Beispiel Nr.) | IC₅₀ [nM] |
| 1 | 6 |
| 1 (3) | 6 |
| 1(4) | 9 |
| 1(6) | 2 |
| 1 (7) | 5 |
| 1 (12) | 2 |
| 1(21) | 2 |
| 1 (26) | 2 |
| 1 (30) | 2 |
| 1(31) | 3 |
| 1 (38) | 1 |
| 1 (39) | 2 |

Die erfindungsgemäß hergestellten Verbindungen sind gut verträglich, da beispielsweise nach oraler Gabe von 10 mg/kg der Verbindung des Beispiels 1 (30) an Ratten keine Änderungen im Verhalten der Tiere beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ 1 und Typ 2, Prädiabetes, Verminderung der Glukosetoleranz oder Veränderungen im Nüchternblutzucker, diabetische Komplikationen (wie z.B. Retinopathie, Nephropathie oder Neuropathien), metabolische Azidose oder Ketose, reaktiver Hypoglykämie, Insulinresistenz, Metabolischem Syndrom, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Weiterhin sind die erfindungsgemäßen Verbindungen zur Behandlung entzündlicher Erkrankungen der Atemwege einsetzbar. Ebenso sind sie zur Prävention und Therapie von chronischen entzündlichen Darmerkrankungen wie z.B. Reizdarmsyndrom (IBS), Morbus Crohn oder Colitis ulcerosa ebenso wie bei Pankreatitis geeignet. Des weiteren wird erwartet, daß sie bei jeglicher Art von Verletzung oder Beeinträchtigung im Gastrointestinaltrakt eingesetzt werden können wie auch z.B. bei Kolitiden und Enteriden. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Auf der anderen Seite sind diese Substanzen geeignet, die Motilität der Spermien zu beeinflussen und sind damit als Kontrazeptiva zur Verwendung beim Mann einsetzbar. Des weiteren sind die Substanzen geeignet, Mangelzustände von Wachstumshormon, die mit Minderwuchs einhergehen, zu beeinflussen, sowie bei allen Indikationen sinnvoll eingesetzt werden können, bei denen Wachstumshormon verwendet werden kann. Die erfindungsgemäßen Verbindungen sind auf Grund ihrer Hemmwirkung gegen DPP IV auch geeignet zur Behandlung von verschiedenen Autoimmunerkrankungen wie z.B. rheumatoide Arthritis, Multiple Sklerose, Thyreoditiden und Basedow'scher Krankheit etc.. Darüberhinaus können sie eingesetzt werden bei viralen Erkrankungen wie auch z.B. bei HIV Infektionen, zur Stimulation der Blutbildung, bei benigner Prostatahyperplasie, bei Gingivitiden, sowie zur Behandlung von neuronalen Defekten und neurdegenerativen Erkrankungen wie z.B. Morbus Alzheimer. Beschriebene Verbindungen sind ebenso zu verwenden zur Therapie von Tumoren, insbesondere zur Veränderung der Tumorinvasion wie auch Metastatisierung, Beispiele hier sind die Anwendung bei T-Zell Lymphomen, akuter lymphoblastischer Leukämie, zellbasierende Schilddrüsenkarzinome, Basalzellkarzinome oder Brustkarzinome. Weitere Indikationen sind Schlaganfall, Ischämien verschiedenster Genese, Morbus Parkinson und Migräne. Darüberhinaus sind weitere Indikationsgebiete follikuläre und epidermale Hyperkeratosen, erhöhte Keratinozytenproliferation, Psoriasis, Enzephalomyelitiden, Glomerulonephritiden, Lipodystrophien, sowie psychosomatische, depressive und neuropsychiatrische Erkrankungen verschiedenster Genese.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. GI 262570) und -Antagonisten, PPAR-gammalalpha Modulatoren (z.B. KRP 297), PPAR-gamma/alpha/delta Modulatoren, AMPK-Aktivatoren, ACC1 und ACC2 Inhibitoren, DGAT-Inhibitoren, SMT3-Rezeptor-Agonisten, 11ß-HSD-Inhibitoren, FGF19-Agonisten oder -Mimetika, alpha-Glucosidasehemmer (z.B. Acarbose, Voglibose), andere DPPIV Inhibitoren, alpha2-Antagonisten, Insulin und Insulinanaloga, GLP-1 und GLP-1 Analoga (z.B. Exendin-4) oder Amylin. Daneben sind Kombinationen mit SGLT2-Inhibitoren wie T-1095 oder KGT-1251 (869682), Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und-Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha Agonisten, PPAR-delta Agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder LXRalpha Antagonisten, LXRbeta Agonisten oder LXRalpha/beta Regulatoren oder Wirkstoffe zur Behandlung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder β₃-Agonisten wie SB-418790 oder AD-9677 ebenso wie Agonisten des 5HT2c Rezeptors möglich.

Daneben ist eine Kombination mit Medikamenten zur Beeinflussung des Bluthochdrucks wie z.B. All Antagonisten oder ACE Inhibitoren, Diuretika, β-Blocker, Ca-Antagonisten und anderen oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

### Herstellung der Ausgangsverbindungen

### Beispiel I

### 1-[(4-Phenylamino-chinazolin-2-yl)methyl]-3-methyl-7-(2-butin-1-yl)-8-[3-(tert.-butyloxycarbonylamino)-piperidin-1-yl]-xanthin

Ein Gemisch aus 416 mg 3-Methyl-7-(2-butin-1-yl)-8-[3-(tert.-butyloxycarbonylamino)-piperidin-1-yl]-xanthin und 456 mg Casiumcarbonat in 4 ml N,N-Dimethylformamid wird bei 80°C für 10 Minuten gerührt, dann werden 324 mg 2-Chlormethyl-4-phenylamino-chinazolin zugegeben und das Reaktionsgemisch wird zwei Stunden bei 80°C gerührt. Dann werden erneut 50 mg Cäsiumcarbonat und 50 mg Chlormethyl-4-phenylamino-chinazolin zugegeben und das Gemisch wird weitere 1.5 Stunden bei 80°C gerührt. Anschließend wird das Lösungsmittel abdestilliert und der Rückstand zwischen Wasser und Essigester verteilt. Die organische Phase wird mit verdünnter Zitronensäure, Wasser und gesättigter Natriumchloridlösung gewaschen, über Magnesiumsulfat getrocknet und eingeengt. Das Rohprodukt wird chromatographisch über eine Kieselgelsäule mit Essigester/Petrolether (8:2 auf 10:0) als Laufmittel gereinigt.
Ausbeute: 425 mg (65 % der Theorie)
R_{f}-Wert: 0.33 (Kieselgel, Essigester)
Massenspektrum (ESI⁺): m/z = 650 [M+H]⁺

Analog Beispiel I werden folgende Verbindungen erhalten:
(1) 1-[(4-Benzylamino-chinazolin-2-yl)methyl]-3-methyl-7-(2-butin-1-yl)-8-[3-(tert.-butyloxycarbonylamino)-piperidin-1-yl]-xanthin
   R_{f}-Wert: 0.20 (Kieselgel, Essigester)
   Massenspektrum (ESI⁺): m/z = 664 [M+H]⁺
(2) 1-[(2-Methyl-chinolin-4-yl)methyl]-3-methyl-7-(2-butin-1-yl)-8-[(R)-3-(tert.-butyloxycarbonylamino)-piperidin-1-yl]-xanthin
   R_{f}-Wert: 0.80 (Kieselgel, Methylenchlorid/Methanol = 9:1)
   Massenspektrum (ESI⁺): m/z = 572 [M+H]⁺
(3) 1-[(3-Cyano-naphthalin-1-yl)methyl]-3-methyl-7-(2-butin-1-yl)-8-[(*R*)-3-(tert.-butyloxycarbonylamino)-piperidin-1-yl]-xanthin
   R_{f}-Wert: 0.67 (Kieselgel, Methylenchlorid/Methanol = 95:5)
   Massenspektrum (ESI⁺): m/z = 582 [M+H]⁺
(4) 1-[(2-Phenyl-chinazolin-4-yl)methyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Essigester = 1:1)
   Massenspektrum (ESI⁺): m/z = 635 [M+H]⁺
(5) 1-[(4-Cyano-isochinolin-1-yl)methyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Methanol/konz. wässriges Ammoniak = 90:10:1)
   Massenspektrum (ESI⁺): m/z = 583 [M+H]⁺
(6) 1-[(4-Cyano-chinolin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 583 [M+H]⁺
(7) 1-[2-(3-Cyclopropyloxy-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Essigester = 1:1)
   Massenspektrum (ESI⁺): m/z = 591 [M+H]⁺
(8) 1-[2-(3-Cyclopropylmethoxy-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.65 (Kieselgel, Methylenchlorid/Essigester = 1:1)
   Massenspektrum (ESI⁺): m/z = 605 [M+H]⁺
(9) 1-[2-(3-Cyclobutyloxy-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.85 (Kieselgel, Methylenchlorid/Essigester = 1:1)
   Massenspektrum (ESI⁺): m/z = 605 [M+H]⁺
(10) 1-[(1-Cyano-isochinolin-3-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 583 [M+H]⁺
(11) 1-[(2,4-Methoxy-naphthalin-1-yl)methyl]-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester)
   Massenspektrum (ESI⁺): m/z = 617 [M+H]⁺
(12) 1-[(2,3-Dimethyl-chinoxalin-6-yl)methyl]-3-methyl-7-(2-butin-1-yl)-8-[(*R*)-3-(tert.-butyloxycarbonylamino)-piperidin-1-yl]-xanthin
   R_{f}-Wert: 0.50 (Kieselgel, Essigester)
   Massenspektrum (ESI⁺): m/z = 587 [M+H]⁺
(13) 1-[(6-Nitro-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.45 (Kieselgel, Essigester/Petrolether = 7:3)
   Massenspektrum (ESI⁺): m/z = 603 [M+H]⁺
(14) 1-[(Chinoxalin-5-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 559 [M+H]⁺
(15) 1-[(6-Methoxy-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.65 (Kieselgel, Essigester)
   Massenspektrum (ESI⁺): m/z = 588 [M+H]⁺
(16) 1-[(6-Phenyl-pyridin-2-yl)methyl]-3-methyl-7-(2-butin-1-yl)-8-[(*R*)-3-(tert.-butyloxycarbony(amino)-piperidin-1-yl]-xanthin
   R_{f}-Wert: 0.43 (Kieselgel, Methylenchlorid/Methanol = 96:4)
   Massenspektrum (ESI⁺): m/z = 584 [M+H]⁺
(17) 1-{[(4-(Pyridin-2-yl)-isochinolin-1-yl]methyl}-3-methyl-7-(2-butin-1-yl)-8-[(*R*)-3-(tert.-butyloxycarbonylamino)-piperidin-1-yl]-xanthin
(18) 1-[(7-Fluor-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.24 (Kieselgel, Essigester/Petrolether = 1:1)
   Massenspektrum (ESI⁺): m/z = 576 [M+H]⁺
(19) 1-[(8-Nitro-chinolin-2-yl)methyl]-3-methyl-7-(2-butin-1-yl)-8-[(-*R*)-3-(tert.-butyloxycarbonylamino)-piperidin-1-yl]-xanthin
   R_{f}-Wert: 0.63 (Kieselgel, Methylenchlorid/Methanol = 95:5)
   Massenspektrum (ESI⁺): m/z = 603 [M+H]⁺
(20) 1-[(6-Fluor-chinolin-2-yl)methyl]-3-methyl-7--(2-butin-1-yl)-8-[(*R*)-3-(tert.-butyloxycarbonylamino)-piperidin-1-yl]-xanthin
   R_{f}-Wert: 0.47 (Kieselgel, Methylenchlorid/Methanol = 95:5)
   Massenspektrum (ESI⁺): m/z = 576 [M+H]⁺
(21) 1-[2-Oxo-2-(2-brom-phenyl)-ethyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Essigester = 1:1)
   Massenspektrum (ESI⁺): m/z = 613, 615 [M+H]⁺
(22) 1-Cyanomethyl-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 456 [M+H]⁺
(23) 1-[(4-Methoxy-chinolin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 588 [M+H]⁺
(24) 1-[(2-Phenyl-pyrimidin-4-yl)methyl]-3-methyl-7-(2-butin-1-yl)-8-[(*R*)-3-(tert.-butyloxycarbonylamino)-piperidin-1-yl]-xanthin
   R_{f}-Wert: 0.39 (Kieselgel, Methylenchlorid/Methanol = 96:4)
   Massenspektrum (ESI⁺): m/z = 585 [M+H]⁺
(25) 1-[([1,5]Naphthyridin-3-yl)methyl]-3-methyl-7-(2-butin-1-yl)-8-[(*R*)-3-(tert.-butyloxycarbonylamino)-piperidin-1-yl]-xanthin
   R_{f}-Wert: 0.28 (Kieselgel, Essigester)
   Massenspektrum (ESI⁺): m/z = 559 [M+H]⁺
(26) 1-[(3-Cyano-4-methyl-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Essigester =1:1)
   Massenspektrum (ESI⁺): m/z = 597 [M+H]⁺
(27) 1-[(4,5-Dimethyl-chinazolin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 587 [M+H]⁺
(28) 1-[(5-Cyano-chinolin-2-yl)methyl]-3-methyl-7-(2-butin-1-yl)-8-[(R)-3-(tert.-butyloxycarbonylamino)-piperidin-1-yl]-xanthin
   R_{f}-Wert: 0.42 (Kieselgel, Petrolether/Essigester = 1:2)
   Massenspektrum (ESI⁺): m/z = 583 [M+H]⁺
(29) 1-[(3-Cyano-chinolin-2-yl)methyl]-3-methyl-7-(2-butin-1-yl)-8-[(R)-3-(tert.-butyloxycarbonylamino)-piperidin-1-yl]-xanthin
   R_{f}-Wert: 0.80 (Kieselgel, Methylenchlorid/Essigester= 1:1)
(30) 1-[(4-Phenyl-pyrimidin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.46 (Kieselgel, Essigester)
(31) 1-[2-(2-Nitro-phenyl)-2-oxo-ethyl]-3-methyl-7-(2-butin-1-yl)-8-[(*R*)-3-(tert.-butyloxycarbony)amino)-piperidin-1-yl]-xanthin
   R_{f}-Wert: 0.75 (Kieselgel, Essigester)
   Massenspektrum (ESI⁺): m/z = 580 [M+H]⁺
(32) 1-[(1,4-Dicyano-naphthalin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.54 (Kieselgel, Methylenchlorid/Methanol = 95:5)
   Massenspektrum (ESI⁺): m/z = 607 [M+H]⁺
(33) 1-[(6,7-Dimethoxy-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.36 (Kieselgel, Essigester)
   Massenspektrum (ESI⁺): m/z = 618 [M+H]⁺
(34) 1-[(Chinazolin-6-yl)methyl]-3-methyl-7-(2-butin-1-yl)-8-[(*R*)-3-(tert.-butyloxycarbonylamino)-piperidin-1-yl]-xanthin
   R_{f}-Wert: 0.20 (Kieselgel, Essigester)
   Massenspektrum (ESI⁺): m/z = 559 [M+H]⁺
(35) 1-[(4-Cyano-chinazolin-2-yl)methyl]-3-methyl-7-(2-butin-1-yl)-8-[(R)-3-(tert.-butyloxycarbonylamino)-piperidin-1-yl]-xanthin
   R_{f}-Wert: 0.40 (Kieselgel, Methylenchlorid/Essigester = 7:3)
   Massenspektrum (ESI⁺): m/z = 584 [M+H]⁺
(36) 1-[(Chinazolin-7-yl)methyl]-3-methyl-7-(2-butin-1-yl)-8-[(*R*)-3-(tert.-butyloxycarbonylamino)-piperidin-1-yl]-xanthin
   R_{f}-Wert: 0.20 (Kieselgel, Essigester)
   Massenspektrum (ESI⁺): m/z = 559 [M+H]⁺
(37) 1-(2-Cyano-benzyl)-3-methyl-7-(2-butin-1-yl)-8-[(*R*)-3-(tert.-butyloxycarbonylamino)-piperidin-1-yl]-xanthin
   Rf-Wert: 0.50 (Kieselgel, Methylenchlorid/Essigester = 7:3)
   Massenspektrum (ESI⁺): m/z = 532 [M+H]⁺
(38) 1-(3-Cyano-benzyl)-3-methyl-7-(2-butin-1-yl)-8-[(*R*)-3-(tert.-butyloxycarbonylamino)-piperidin-1-yl]-xanthin
   R_{f}-Wert: 0.58 (Kieselgel, Methylenchlorid/Essigester = 1:1)
   Massenspektrum (ESI⁺): m/z = 532 [M+H]⁺
(39) 1-(4-Cyano-benzyl)-3-methyl-7-(2-butin-1-yl)-8-[(*R*)-3-(tert.-butyloxycarbonylamino)-piperidin-1-yl]-xanthin
   R_{f}-Wert: 0.61 (Kieselgel, Methylenchlorid/Essigester = 1:1)
   Massenspektrum (ESI⁺): m/z = 532 [M+H]⁺
(40) 1-[(Pyridin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 508 [M+H]⁺
(41) 1-Benzyl-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Methylenchlorid/Essigester =1:1)
   Massenspektrum (ESI⁺): m/z = 507 [M+H]⁺
(42) 1-(4-Methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Essigester)
   Massenspektrum (ESI⁺): m/z = 537 [M⁺H]⁺
(43) 1-(2-Chlor-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 541, 543 [M+H]⁺
(44) 1-(2,6-Dicyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 557 [M+H]⁺
(45) 1-(2-Cyano-4-brom-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 610, 612 [M+H]⁺
(46) 1-(3-Fluor-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 525 [M+H]⁺
(47) 1-(3,5-Dimethoxy-benzyl)-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester)
   Massenspektrum (ESI⁺): m/z = 567 [M+H]⁺
(48) 1-(2-Fluor-benzyl)-3-methyl-7-(2-butin-1-yl)-8-[(*R*)-3-(tert.-butyloxycarbonylamino)-piperidin-1-yl]-xanthin
   R_{f}-Wert: 0.85 (Kieselgel, Essigester)
   Massenspektrum (ESI⁺): m/z = 525 [M+H]⁺
(49) 1-[(6-Cyano-pyridin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.60 (Kieselgel, Methylenchlorid/Essigester= 1:1)
   Massenspektrum (ESI⁺): m/z = 533 [M+H]⁺
(50) 1-[(3-Cyano-pyridin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.60 (Kieselgel, Methylenchlorid/Essigester = 1:1)
   Massenspektrum (ESI⁺): m/z = 533 [M+H]⁺
(51) 1-(2-Cyano-3-chlor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 566, 568 [M+H]⁺
(52) 1-(4-Fluor-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 525 [M+H]⁺
(53) 1-(4-Chlor-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 541, 543 [M+H]⁺
(54) 1-(2-Cyano-4-fluor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 550 [M+H]⁺
(55) 1-(3-Cyano-4-fluor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 550 [M+H]⁺
(56) 1-(2-Chlor-4-cyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 566, 568 [M+H]⁺
(57) 1-[(5-Methoxycarbonyl-furan-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 555 [M+H]⁺
(58) 1-(2-Trifluormethyl-4-cyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 600 [M+H]⁺
(59) 1-(3,5-Dicyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 557 [M+H]⁺
(60) 1-(3-Nitro-4-cyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 577 [M+H]⁺
(61) 1-[(2-Cyano-pyridin-3-yl)methyl]-3-methyl-7-(2-butin-1-yl)-8-[(*R*)-3-(tert.-butyloxycarbonylamino)-piperidin-1-yl]-xanthin
   R_{f}-Wert: 0.50 (Kieselgel, Methylenchlorid/Essigester = 1:1)
   Massenspektrum (ESI⁺): m/z = 533 [M+H]⁺
(62) 1-(2-Cyano-4-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Methylenchlorid/Essigester = 1:1)
   Massenspektrum (ESI⁺): m/z = 562 [M+H]⁺
(63) 1-(2-Cyano-5-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Methylenchlorid/Essigester =1:1)
   Massenspektrum (ESI⁺): m/z = 562 [M+H]⁺
(64) 1-(3-Methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 537 [M+H]⁺
(65) 1-(3-Trifluormethyl-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 575 [M+H]⁺
(66) 1-(3,4-Dimethoxy-benzyl)-3-methyl-7-(2-butin-1-yl)-8-[(*R*)-3-(tert.-butyloxycarbonylamino)-piperidin-1-yl]-xanthin
   R_{f}-Wert: 0.65 (Kieselgel, Essigester)
   Massenspektrum (ESI⁺): m/z = 567 [M+H]⁺
(67) 1-(3-Chlor-benzyl)-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 541, 543 [M+H]⁺
(68) 1-(4-Trifluormethyl-benzyl)-3-methyl-7-(2-butin-1-yl)-8-[(*R*)-3-(tert.-butyloxycarbonylamino)-piperidin-1-yl]-xanthin
   R_{f}-Wert: 0.85 (Kieselgel, Essigester)
   Massenspektrum (ESI⁺): m/z = 575 [M+H]⁺
(69) 1-[([2,2']Bipyridinyl-6-yl)methyl]-3-methyl-7-(2-butin-1-yl)-8-[(*R*)-3-(tert.-butyloxycarbonylamino)-piperidin-1-yl]-xanthin
   R_{f}-Wert: 0.53 (Aluminiumoxid, Methylenchlorid/Methanol = 98:2)
   Massenspektrum (ESI⁺): m/z = 585 [M+H]⁺
(70) 1-(3,4-Dimethoxy-6-fluor-benzyl)-3-methyl-7-(2-butin-1-yl)-8-[(*R*)-3-(tert.-butyloxycarbonylamino)-piperidin-1-yl]-xanthin
   R_{f}-Wert: 0.65 (Kieselgel, Essigester)
   Massenspektrum (ESI⁺): m/z = 585 [M+H]⁺
(71) 1-[(6-Fluor-pyridin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 526 [M+H]⁺
(72) 1-[(5-Cyano-6-methoxy-pyridin-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 563 [M+H]⁺
(73) 1-(2,6-Difluor-benzyl)-3-methyl-7-(2-butin-1-yl)-8-[(*R*)-3-(tert.-butyloxycarbonylamino)-piperidin-1-yl]-xanthin
   R_{f}-Wert: 0.62 (Kieselgel, Essigester)
   Massenspektrum (ESI⁺): m/z = 543 [M+H]⁺
(74) 1-(3-Trifluormethoxy-benzyl)-3-methyl-7-(2-butin-1-yl)-8-[(*R*)-3-(tert.-butyloxycarbonylamino)-piperidin-1-yl]-xanthin
   R_{f}-Wert: 0.67 (Kieselgel, Essigester)
   Massenspektrum (ESI⁺): m/z = 591 [M+H]⁺
(75) 1-(4-Trifluormethoxy-benzyl)-3-methyl-7-(2-butin-1-yl)-8-[(*R*)-3-(tert.-butyloxycarbonylamino)-piperidin-1-yl]-xanthin
   R_{f}-Wert: 0.62 (Kieselgel, Essigester)
   Massenspektrum (ESI⁺): m/z = 591 [M+H]⁺
(76) 1-[(2-Cyano-pyridin-4-yl)methyl]-3-methyl-7-(2-butin-1-yl)-8-[(*R*)-3-(tert.-butyloxycarbonylamino)-piperidin-1-yl]-xanthin
   R_{f}-Wert: 0.55 (Kieselgel, Methylenchlorid/Essigester = 1:1)
   Massenspektrum (ESI⁺): m/z = 533 [M+H]⁺
(77) 1-[(5-Cyano-pyridin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.55 (Kieselgel, Methylenchlorid/Essigester = 1:1)
   Massenspektrum (ESI⁺): m/z = 533 [M+H]⁺
(78) 1-[(Pyrimidin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.60 (Kieselgel, Essigester/Methanol = 9:1)
   Massenspektrum (ESI⁺): m/z = 509 [M+H]⁺
(79) 1-[(4-Methyl-pyrimidin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.60 (Kieselgel, Essigester/Methanol = 9:1)
   Massenspektrum (ESI⁺): m/z = 523 [M+H]⁺
(80) 1-[(4,6-Dimethyl-pyrimidin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester/Methanol = 9:1)
   Massenspektrum (ESI⁺): m/z = 537 [M+H]⁺
(81) 1-[(Chinoxalin-6-yl)methyl]-3-methyl-7-(2-butin-1-yl)-8-brom-xanthin
   R_{f}-Wert: 0.55 (Kieselgel, Essigester)
   Massenspektrum (ESI⁺): m/z = 439, 441 [M+H]⁺
(82) 1-(3-Fluor-4-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester)
   Massenspektrum (ESI⁺): m/z = 555 [M+H]⁺
(83) 1-(3,4-Difluor-benzyl)-3-methyl-7-(2-butin-1-yl)-8-[(*R*)-3-(tert.-butyloxycarbonylamino)-piperidin-1-yl]-xanthin
   R_{f}-Wert: 0.75 (Kieselgel, Essigester)
   Massenspektrum (ESI⁺): m/z = 543 [M+H]⁺
(84) 1-(2-Fluor-5-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.55 (Kieselgel, Essigester/Petrolether = 3:2)
   Massenspektrum (ESI⁺): m/z = 555 [M+H]⁺
(85) 1-(2-Fluor-3-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.48 (Kieselgel, Essigester/Petrolether = 3:2)
   Massenspektrum (ESI⁺): m/z = 555 [M+H]⁺
(86) 1-[(4-Cyano-isochinolin-3-yl)methyl]-3-methyl-7-(2-butin-1-yl)-8-[(*R*)-3-(tert.-butyloxycarbonylamino)-piperidin-1-yl]-xanthin
   R_{f}-Wert: 0.55 (Kieselgel, Methylenchlorid/Methanol= 95:5)
   Massenspektrum (ESI⁺): m/z = 583 [M+H]⁺
(87) 1-(2-Fluor-4-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.48 (Kieselgel, Essigester/Petrolether = 1:1)
   Massenspektrum (ESI⁺): m/z = 555 [M+H]⁺
(88) 1-[(Furan-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 497 [M+H]⁺
(89) 1-(3,4-Dicyano-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 557 [M+H]⁺
(90) 1-(4-Cyano-2-fluor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 550 [M+H]⁺
(91) (1-(2-Cyano-5-fluor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 550 [M+H]⁺
(92) 1-[(5-Formyl-furan-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 525 [M+H]⁺
(93) 1-(2-Cyano-6-fluor-benzyl)-3-methyl-7-(2-butin-1-yl)-8-[(*R*)-3-(tert.-butyloxycarbonylamino)-piperidin-1-yl]-xanthin
(94) 1-(4-Cyano-3-fluor-benzyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 550 [M+H]⁺
(95) 1-(2-Cyano-3-methoxy-benzyl)-3-methyl-7-(2-butin-1-yl)-8-brom-xanthin
   R_{f}-Wert: 0.85 (Kieselgel, Essigester)
   Massenspektrum (ESI⁺): m/z = 442, 444 [M+H]⁺
(96) 1-[(8-Cyano-chinolin-7-yl)methyl]-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester)
   Massenspektrum (ESI⁺): m/z = 583 [M+H]⁺
(97) 1-[(4-Cyano-pyridin-3-yl)methyl]-3-methyl-7-(2-butin-1-yl)-8-[(*R*)-3-(tert.-butyloxycarbonylamino)-piperidin-1-yl]-xanthin
   R_{f}-Wert: 0.40 (Kieselgel, Essigester/Cyclohexan = 3:1)
   Massenspektrum (ESI⁺): m/z = 533 [M+H]⁺
(98) 1-[(8-Cyano-chinolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.40 (Kieselgel, Essigester/Petrolether = 4:1)
   Massenspektrum (ESI⁺): m/z = 583 [M+H]⁺
(99) 1-[(1-Methyl-1H-benzotriazol-5-yl)methyl]-3-methyl-7-(2-butin-1-yl)-8-[(*R)*-3-(tert.-butyloxycarbonylamino)-piperidin-1-yl]-xanthin
   R_{f}-Wert: 0.30 (Kieselgel, Methylenchlorid/Essigester = 1:1)
   Massenspektrum (ESI⁺): m/z = 562 [M+H]⁺
(100) 1-[(3-Cyano-pyridin-4-yl)methyl]-3-methyl-7-(2-butin-1-yl)-8-[(*R*)-3-(tert.-butyloxycarbonylamino)-piperidin-1-yl]-xanthin
   R_{f}-Wert: 0.30 (Kieselgel, Methylenchlorid/Essigester = 1:1)
   Massenspektrum (ESI⁺): m/z = 533 [M+H]⁺
(101) 1-[(3-Cyano-pyridin-2-yl)methyl]-3-methyl-7-(2-butin-1-yl)-8-brom-xanthin
   Massenspektrum (ESI⁺): m/z = 413, 415 [M+H]⁺
(102) 1-[(4-Cyano-benzo[1,3]dioxol-5-yl)methyl]-3-methyl-7-(2-butin-1-yl)-8-[(*R*)-3-(tert.-butyloxycarbonylamino)-piperidin-1-yl]-xanthin
   R_{f}-Wert: 0.80 (Kieselgel, Essigester)
   Massenspektrum (ESI⁺): m/z = 576 [M+H]⁺

### Beispiel II

### 3-Methyl-7-(2-butin-1-yl)-8-[(R)-3-(tert.-butyloxycarbonylamino)-piperidin-1-yl]-xanthin

Zu 15.00 g 3-Methyl-7-(2-butin-1-yl)-8-brom-xanthin und 16.00 g Kaliumcarbonat in 100 ml Dimethylsulfoxid werden 11.00 g (R)-3-tert.-Butyloxycarbonylamino-piperidin gegeben und die dicke hellbeige Suspension wird vier Stunden mit einem mechanischen Rührer bei ca. 114°C gerührt. Dann werden nochmals 900 mg (R)-3-tert.-Butyloxycarbonylamino-piperidin, gelöst in 10 ml Dimethylsulfoxid, zum Reaktionsgemisch gegeben und dieses wird weitere zwei Stunden bei 114°C gerührt.

Nach Abkühlung auf Raumtemperatur wird das Reaktionsgemisch mit reichlich Wasser verdünnt. Der entstandene Niederschlag wird gründlich verrieben, bis keine Klumpen mehr vorhanden sind, und absaugt. Der helle Feststoff wird erneut mit Wasser aufgeschlämmt, abgesaugt, mit Wasser und Diethylether nachgewaschen und im Umlufttrockenschrank bei 60°C getrocknet.
Ausbeute: 19.73 g (94 % der Theorie)
R_{f}-Wert: 0.64 (Kieselgel, Essigester)
Massenspektrum (ESI⁺): m/z = 417 [M+H]⁺

Analog Beispiel II wird folgende Verbindung erhalten:
(1) 3-Methyl-7-(2-butin-1-yl)-8-[(3-(tert.-butyloxycarbonylamino)-piperidin-1-yl]-xanthin
   Schmelzpunkt: 235-237°C
   Massenspektrum (ESI⁺): m/z = 417 [M+H]⁺
(2) 1-[(Chinoxalin-6-yl)methyl]-3-methyl-7-(2-butin-1-yl)-8-[(*R*)-3-(tert.-butyloxycarbonylamino)-piperidin-1-yl]-xanthin
   R_{f}-Wert: 0.40 (Kieselgel, Essigester)
   Massenspektrum (ESI⁺): m/z = 559 [M+H]⁺
(3) 1-[(5-Methyl-furan-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 511 [M+H]⁺
(4) 1-(2-Cyano-3-methoxy-benzyl)-3-methyl-7-(2-butin-1-yl)-8-[(*R*)-3-(tert.-butyloxycarbonylamino)-piperidin-1-yl]-xanthin
   R_{f}-Wert: 0.50 (Kieselgel, Essigester)
   Massenspektrum (ESI⁺): m/z = 562 [M+H]⁺
(5) 1-[(3-Cyano-pyridin-2-yl)methyl]-3-methyl-7-(2-butin-1-yl)-8-[(*S*)-3-(tert.-butyloxycarbonylamino)-piperidin-1-yl]-xanthin
   R_{f}-Wert: 0.50 (Kieselgel, Essigester)
   Massenspektrum (ESI⁺): m/z = 533 [M+H]⁺

### Beispiel III)

### 3-Methyl-7-(2-butin-1-yl)-8-brom-xanthin

Zu 30.17 g 3-Methyl-8-brom-xanthin und 27.00 ml Hünigbase in 370 ml N,N-Dimethylformamid werden 17.06 g 1-Brom-2-butin gegeben. Das Reaktionsgemisch wird zwei Stunden bei Raumtemperatur gerührt, dann wird nochmals 1 ml 1-Brom-2-butin nachgesetzt und eine weitere Stunde bei Raumtemperatur gerührt. Zur Aufarbeitung wird das Reaktionsgemisch mit ca. 300 ml Wasser verdünnt. Der entstandene helle Niederschlag wird abgesaugt und mit Wasser nachgewaschen. Der Filterkuchen wird mit wenig Ethanol und Diethylether gewaschen und im Umlufttrockenschrank bei 60°C getrocknet.
Ausbeute: 30.50 g (84 % der Theorie)
R_{f}-Wert: 0.24 (Kieselgel, Methylenchlorid/Methanol = 95:5)
Massenspektrum (ESI⁺): m/z = 297, 299 [M+H]⁺

### Beispiel IV

### 2-Chlormethyl-4-phenylamino-chinazolin

Hergestellt durch Umsetzung von 500 mg 4-Chlor-2-chloromethyl-chinazolin mit 438 mg Anilin in 12 ml Methylenchlorid bei Raumtemperatur.
Ausbeute: 518 mg (82 % der Theorie)
R_{f}-Wert: 0.60 (Kieselgel, Cyclohexan/Essigester = 1:1)
Massenspektrum (ESI⁺): m/z = 270, 272 [M+H]⁺

Analog Beispiel IV wird folgende Verbindung erhalten:
(1) 2-Chlormethyl-4-benzylamino-chinazolin
   R_{f}-Wert: 0.60 (Kieselgel, Cyclohexan/Essigester 1:1)
   Massenspektrum (ESI⁺): m/z = 284, 286 [M+H]⁺

### Beispiel V

### 1-Brommethyl-4-cyano-isochinolin

Hergestellt durch Bromierung von 1-Methyl-4-cyano-isochinolin mit N-Bromsuccinimid in Gegenwart von Azobisisobutyronitril in Tetrachlorkohlenstoff bei 80°C.

R_{f}-Wert: 0.51 (Kieselgel, Methylenchlorid)
Massenspektrum (EI): m/z = 246, 248 [M]⁺
Analog Beispiel V werden folgende Verbindungen erhalten:
(1) 2-Brommethyl-4-cyano-chinolin
   Massenspektrum (ESI⁺): m/z = 247,249 [M+H]⁺
(2) 3-Brommethyl-1-cyano-isochinolin
   Massenspektrum (ESI⁺): m/z = 247, 249 [M+H]⁺
(3) 1-Brommethyl-4-(pyridin-2-yl)-isochinolin
   R_{f}-Wert: 0.47 (Kieselgel, Methylenchlorid/Methanol = 9:1)
(4) 2-Brommethyl-4-methoxy-chinolin
   Massenspektrum (ESI⁺): m/z = 252, 254 [M+H]⁺
(5) 3-Brommethyl-[1,5]naphthyridin
   Massenspektrum (ESI⁺): m/z = 223, 225 [M+H]⁺
(6) 2-Brommethyl-5-cyano-chinolin
   R_{f}-Wert: 0.28 (Kieselgel, Petrolether/Essigester = 5:1)
   Massenspektrum (ESI⁺): m/z = 247, 249 [M+H]⁺
(7) 2-Brommethyl-3-cyano-chinolin
   R_{f}-Wert: 0.65 (Kieselgel, Cyclohexan/Essigester = 3:1)
(8) 2-Brommethyl-4-phenyl-pyrimidin
   R_{f}-Wert: 0.88 (Kieselgel, Essigester)
   Massenspektrum (ESI⁺): m/z = 249, 251 [M+H]⁺
(9) 2-Brommethyl-1,4-dicyano-naphthalin
   R_{f}-Wert: 0.48 (Kieselgel, Petrolether/Essigester = 9:1)
   Massenspektrum (EI⁺): m/z = 270, 272 [M]⁺
(10) 2-Brommethyl-6,7-dimethoxy-chinolin
   R_{f}-Wert: 0.70 (Kieselgel, Methylenchlorid/Methanol = 95:5)
   Massenspektrum (ESI⁺): m/z = 282, 284 [M+H]⁺
(11) 2-Brommethyl-4-cyano-chinazolin
   R_{f}-Wert: 0.85 (Kieselgel, Methylenchlorid/Methanol = 99:1)
   Massenspektrum (EI⁺): m/z = 247, 249 [M]⁺
(12) 7-Brommethyl-chinazolin
   R_{f}-Wert: 0.15 (Kieselgel, Methylenchlorid/Methanol = 99:1)
   Massenspektrum (ESI⁺): m/z = 223, 225 [M+H]⁺
(13) 2-Trifluormethyl-4-cyano-benzylbromid
(14) 2-Brommethyl-5-cyano-6-methoxy-pyridin Massenspektrum (ESI⁺): m/z = 227, 229 [M+H]⁺
(15) 3-Brommethyl-4-cyano-isochinolin
   R_{f}-Wert: 0.43 (Kieselgel, Petrolether/Essigester = 7:3)
(16) 7-Brommethyl-8-cyano-chinolin
   R_{f}-Wert: 0.25 (Kieselgel, Petrolether/Essigester = 7:3)
   Massenspektrum (ESI⁺): m/z = 247, 249 [M+H]⁺
(17) 2-Brommethyl-8-cyano-chinolin
   R_{f}-Wert: 0.75 (Kieselgel, Methylenchlorid/Methanol = 99:1)
   Massenspektrum (ESI⁺): m/z = 247, 249 [M+H]⁺

### Beispiel VI

### 2-Brom-1-(3-cyclopropyloxy-phenyl)-ethanon

Hergestellt durch Bromierung von 1-(3-Cyclopropyloxy-phenyl)-ethanon mit Phenyltrimethylammoniumtribromid in Methylenchlorid unter Rückfluss.
R_{f}-Wert: 0.75 (Kieselgel, Cyclohexan/Essigester = 3:1)
Massenspektrum (ESI⁺): m/z = 255, 257 [M+H]⁺

Analog Beispiel VI werden folgende Verbindungen erhalten:
(1) 2-Brom-1-(3-cyclopropylmethoxy-phenyl)-ethanon
   R_{f}-Wert: 0.70 (Kieselgel, Cyclohexan/Essigester = 3:1)
(2) 2-Brom-1-(3-cyclobutyloxy-phenyl)-ethanon
   R_{f}-Wert: 0.70 (Kieselgel, Cyclohexan/Essigester = 3:1)

### Beispiel VII

### 1-(3-Cyclopropyloxy-phenyl)-ethanon

Hergestellt durch Umsetzung von 3-Hydroxyacetophenon mit Bromcyclopropan in Gegenwart von Kaliumiodid und Kalium-tert.-butylat in N,N-Dimethylformamid in der Mikrowelle bei 220°C.
R_{f}-Wert: 0.65 (Kieselgel, Cyclohexan/Essigester = 3:1)
Massenspektrum (ESI⁺): m/z = 177 [M+H]⁺

Analog Beispiel VII werden folgende Verbindungen erhalten:
(1) 1-(3-Cyclopropylmethoxy-phenyl)-ethanon
   R_{f}-Wert: 0.70 (Kieselgel, Cyclohexan/Essigester = 3:1)
   Massenspektrum (ESI⁺): m/z = 191 [M+H]⁺
(2) 1-(3-Cyclobutyloxy-phenyl)-ethanon
   R_{f}-Wert: 0.65 (Kieselgel, Cyclohexan/Essigester = 3:1)
   Massenspektrum (ESI⁺): m/z = 191 [M+H]⁺

### Beispiel VIII

### 1-Chlormethyl-2,4-dimethoxy-naphthalin

Hergestellt durch Chlorierung von 1-Hydroxymethyl-2,4-dimethoxy-naphthalin mit Thionylchlorid in Methylenchlorid bei Raumtemperatur.
R_{f}-Wert: 0.78 (Kieselgel, Cyclohexan/Essigester = 1:1)
Massenspektrum (EI): m/z = 236, 238 [M]⁺

### Beispiel IX

### 1-Hydroxymethyl-2,4-dimethoxy-naphthalin

Hergestellt durch Reduktion von 2,4-Dimethoxy-naphthalin-1-carboxaldehyd mit Natriumborhydrid in einem Gemisch aus Dioxan und Wasser (3:1) bei Raumtemperatur.
R_{f}-Wert: 0.48 (Kieselgel, Cyclohexan/Essigester = 1:1)

### Beispiel X

### 1-[(6-Amino-chinolin-2-yl)methyl]-3-methyl-7-(2-butin-1-yl)-8-[(R)-3-(tert.-butyloxy-carbonylamino)-piperidin-1-yl]-xanthin

Hergestellt durch Behandlung von 1-[(6-Nitro-chinolin-2-yl)methyl]-3-methyl-7-(2-butin-1-yl)-8-[(*R*)-3-(tert.-butyloxycarbonylamino)-piperidin-1-yl]-xanthin mit Natriumdithionit in einem Gemisch aus Ethanol/Wasser (5:2) bei 55-60°C.
R_{f}-Wert: 0.40 (Kieselgel, Methylenchlorid/Methanol = 95:5)
Massenspektrum (ESI⁺): m/z = 573 [M+H]⁺

### Beispiel XI

### 1-Methyl-4-(pyridin-2-yl)-isochinolin

Hergestellt durch Umsetzung von 4-Brom-1-methyl-isochinolin mit Lithium-Triiisopropoxy-2-pyridinyl-boronat in Gegenwart von Tetrakis(triphenylphosphin)-palladium, Triphenylphosphin, Natriumcarbonat und Kupfer(I)iodid in 1,4-Dioxan unter Rückfluss.
R_{f}-Wert: 0.22 (Kieselgel, Methylenchlorid/Methanol = 95:5)
Massenspektrum (ESI⁺): m/z = 221 [M+H]⁺

### Beispiel XII

### 1-[(8-Amino-chinolin-2-yl)methyl]-3-methyl-7-(2-butin-1-yl)-8-[(R)-3-(tert.-butyloxy-_ carbonylamino)-piperidin-1-yl]-xanthin

Hergestellt durch Behandlung von 1-[(8-Nitro-chinolin-2-yl)methyl]-3-methyl-7-(2-butin-1-yl)-8-[(*R*)-3-(tert.-butyloxycarbonylamino)-piperidin-1-yl]-xanthin mit Eisenpulver in einem Gemisch aus Eisessig, Ethanol und Wasser (2:20:5) unter Rückfluss. R_{f}-Wert: 0.50 (Kieselgel, Methylenchlorid/Methanol = 95:5)
Massenspektrum (ESI⁺): m/z = 573 [M+H]⁺

### Beispiel XIII

### 1-{2-Oxo-2-[2-(pyridin-3-yl)-phenyl]-ethyl}-3-methyl-7-(2-butin-1-yl)-8-[(R)-3-(tert.-butyloxycarbonylamino)-piperidin-1-yl]-xanthin

Hergestellt durch Umsetzung von 1-[2-Oxo-2-(2-brom-phenyl)-ethyl]-3-methyl-7-(2-butin-1-yl)-8-[(*R*)-3-(tert.-butyloxycarbonylamino)-piperidin-1-yl]-xanthin mit Pyridin-3-boronsäure in Gegenwart von Tetrakis(triphenylphosphin)palladium, Tetra-n-butylammoniumbromid und Natriumcarbonat in einem Gemisch aus Toluol/Ethanol (1:1) bei 105°C.
R_{f}-Wert: 0.55 (Kieselgel, Essigester)
Massenspektrum (ESI⁺): m/z = 612 [M+H]⁺ .

Analog Beispiel XII wird folgende Verbindung erhalten:
(1) 1-{2-Oxo-2-[2-(pyridin-4-yl)-phenyl]-ethyl}-3-methyl-7-(2-butin-1-yl)-8-[(*R*)-3-(tert.-butyloxycarbonylamino)-piperidin-1-yl]-xanthin
   (Die Umsetzung erfolgt mit 4-(4,4,5,5-Tetramethyl-[1,3,2]dioxaborolan-2-yl)-pyridin.)
   R_{f}-Wert: 0.40 (Kieselgel, Essigester)
   Massenspektrum (ESI⁺): m/z = 612 [M+H]⁺

### Beispiel XIV

### 1-[(4-Ethyl-chinazolin-2-yl)methyl]-3-methyl-7-(2-butin-1-yl)-8-[(R)-3-(tert.-butyloxycarbonylamino)-piperidin-1-yl]-xanthin

Hergestellt durch Behandlung von 1-Cyanomethyl-3-methyl-7-(2-butin-1-yl)-8-[(*R*)-3-(tert.-butyloxycarbonylamino)-piperidin-1-yl]-xanthin mit Kalium-tert.-butylat in Methanol und anschließende Umsetzung des entstandenen Iminoesters mit 2-Aminopropiophenon in Gegenwart von Eisessig.
R_{f}-Wert: 0.60 (Kieselgel, Essigester)
Massenspektrum (ESI⁺): m/z = 587 [M+H]⁺
Analog Beispiel XIV wird folgende Verbindung erhalten:
(1) 1-[(4-Cyclopropyl-chinazolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.70 (Kieselgel, Essigester)
   Massenspektrum (ESI⁺): m/z = 599 [M+H]⁺

### Beispiel XV

### 2-Chlormethyl-3-cyano-4-methyl-chinolin

Hergestellt durch Umsetzung von 3-Cyano-2,4-dimethyl-1-oxy-chinolin mit Benzosulfonsäurechlorid in Toluol bei 80°C.
R_{f}-Wert: 0.55 (Kieselgel, Cyclohexan/Essigester = 2:1)
Massenspektrum (ESI⁺): m/z =217,219 [M+H]⁺

### Beispiel XVI

### 3-Cyano-2,4-dimethyl-1-oxy-chinolin

Hergestellt durch Behandlung von 3-Cyano-2,4-dimethyl-chinolin mit wässriger Wasserstoffperoxidlösung (35 %) in Eisessig bei 60°C.
R_{f}-Wert: 0.35 (Kieselgel, Essigester)
Massenspektrum (ESI⁺): m/z = 199 [M+H]⁺

### Beispiel XVII

### 2-Chlormethyl-4,5-dimethyl-chinazolin

Hergestellt durch Umsetzung von 1-(2-Amino-6-methyl-phenyl)-ethanon mit Chloracetonitril in Dioxan unter Einleitung von Chlorwasserstoff bei 30-38°C. Massenspektrum (ESI⁺): m/z = 207, 209 [M+H]⁺

### Beispiel XVIII

### 1-[(2-Methyl-chinazolin-4-yl)methyl]-3-methyl-7-(2-butin-1-yl)-8-[(R)-3-(tert.-butyloxycarbonylamino)-piperidin-1-yl]-xanthin

Hergestellt durch Umsetzung von 1-[2-(2-Acetylamino-phenyl)-2-oxo-ethyl]-3-methyl-7-(2-butin-1-yl)-8-[(*R*)-3-(tert.-butyloxycarbonylamino)-piperidin-1-yl]-xanthin mit ethanolischem Ammoniak (6 M) und Ammoniumchlorid im Autoklaven bei 150°C.
R_{f}-Wert: 0.35 (Kieselgel, Essigester)
Massenspektrum (ESI⁺): m/z = 573 [M+H]⁺

### Beispiel XIX

### 1-[2-(2-Acetylamino-phenyl)-2-oxo-ethyl]-3-methyl-7-(2-butin-1-yl)-8-[(R)-3-(tert.-butyloxycarbonylamino)-piperidin-1-yl]-xanthin

Hergestellt durch Umsetzung von 1-[2-(2-Amino-phenyl)-2-oxo-ethyl]-3-methyl-7-(2-butin-1-yl)-8-[(*R*)-3-(tert.-butyloxycarbonylamino)-piperidin-1-yl]-xanthin mit Acetylchlorid in Gegenwart von Pyridin in Methylenchlorid bei Raumtemperatur.
R_{f}-Wert: 0.79 (Kieselgel, Essigester)
Massenspektrum (ESI⁺): m/z = 592 [M+H]⁺

### Beispiel XX

### 1-[2-(2-Amino-phenyl)-2-oxo-ethyl]-3-methyl-7-(2-butin-1-yl)-8-[(R)-3-(tert.-butyloxycarbonylamino)-piperidin-1-yl]-xanthin

Hergestellt durch Reduktion von 1-[2-(2-Nitro-phenyl)-2-oxo-ethyl]-3-methyl-7-(2-butin-1-yl)-8-[(*R*)-3-(tert.-butyloxycarbonylamino)-piperidin-1-yl]-xanthin mit Zinn(II)-chlorid-dihydrat in Tetrahydrofuran bei Raumtemperatur.
R_{f}-Wert: 0.85 (Kieselgel, Essigester)
Massenspektrum (ESI⁺): m/z = 550 [M+H]⁺

### Beispiel XXI

### 1-[(Furan-3-yl)methyl]-3-methyl-7-(2-butin-1-yl)-8-[(R)-3-(tert.-butyloxycarbonylamino)-piperidin-1-yll-xanthin

Ein Gemisch aus 300 mg 3-Methyl-7-(2-butin-1-yl)-8-[(*R*)-3-(tert.-butyloxycarbonylamino)-piperidin-1-yl]-xanthin, 95 µl Furan-3-yl-methanol, 302 mg Triphenylphosphin und 226 µl Diisopropylazodicarboxylat in 4 ml Tetrahydrofuran wird über Nacht bei Raumtemperatur gerührt. Zur Aufarbeitung wird das Reaktionsgemisch mit gesättigter Kaliumcarbonat-Lösung versetzt und mit Essigester extrahiert. Die vereinigten organischen Phasen werden über Magnesiumsulfat getrocknet und eingeengt. Der Kolbenrückstand wird über eine Kieselgelsäule mit Cyclohexan/Essigester (1:1 auf 3:7) chromatographiert.
Ausbeute: 330 mg (92 % der Theorie)
Massenspektrum (ESI⁺): m/z = 497 [M+H]⁺

Analog Beispiel XXI werden folgende Verbindungen erhalten:
(1) 1-[(5-Methyl-furan-2-yl)methyl]-3-methyl-7-(2-butin-1-yl)-8-brom-xanthin
   Massenspektrum (ESI⁺): m/z = 391, 393 [M+H]⁺
(2) 1-[(5-Brom-furan-2-yl)methyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 575, 577 [M+H]⁺

### Beispiel XXII

### 1-[(5-Cyano-furan-2-yl)methyl]-3-methyl-7-(2-butin-1-yl)-8-[(R)-3-(tert.-buloxycarbonylamino)-piperidin-1-yl]-xanthin

Hergestellt durch Umsetzung von 1-[(5-Formyl-furan-2-yl)methyl]-3-methyl-7-(2-butin-1-yl)-8-[(*R*)-3-(tert.-butyloxycarbonylamino)-piperidin-1-yl]-xanthin mit Hydroxylamin-O-sulfonsäure und Pyridin in Toluol unter Rückfluss.

### Beispiel XXIII

### 5-(Methansulfonyloxymethyl)-2-furan-carboxaldehyd

Hergestellt durch Umsetzung von 5-(Hydroxymethyl)-2-furan-carboxaldehyd mit Methansulfonsäurechlorid in Gegenwart von Triethylamin in Methylenchlorid bei Raumtemperatur. Das Rohprodukt wird ohne weitere Reingung weiter umgesetzt.

### Beispiel XXIV

### 2-Chlormethyl-3-cyano-pyridin

Hergestellt aus 2-(Hydroxymethyl)-nicotinamid durch Umsetzung mit Thionylchlorid in Acetonitril und anschliessende Dehydratisierung des so erhaltenen 2-(Chlormethyl)-nicotinamids mit Trifluoressigsäureanhydrid in Gegenwart von Triethylamin in Methylenchlorid.
Alternativ wird die Verbindung auch in einem Schritt durch Erhitzen von 2-(Hydroxymethyl)-nicotinamid mit Phosphoroxychlorid unter Rückfluß erhalten.
R_{f}-Wert: 0.85 (Kieselgel, Methylenchlorid/Methanol = 9:1)
Massenspektrum (ES⁺): m/z = 153, 155 [M+H]⁺

### Beispiel XXV

### 8-Cyano-7-methyl-chinolin

Hergestellt durch Umsetzung von 8-Brom-7-methyl-chinolin mit Zinkcyanid in Gegenwart von Tetrakis(triphenylphosphin)palladium in N-Methylpyrrolidinon unter Schutzgasatmosphäre bei 100-105°C.
R_{f}-Wert: 0.35 (Kieselgel, Petrolether/Essigester = 7:3)
Massenspektrum (ESI⁺): m/z = 169 [M+H]⁺

### Beispiel XXVI

### 2-Methyl-8-cyano-chinolin

Hergestellt durch Umsetzung von 2-Methyl-8-brom-chinolin mit Kupfer(I)cyanid in N-Methylpyrrolidinon unter Schutzgasatmosphäre bei 180°C.
R_{f}-Wert: 0.40 (Kieselgel, Petrolether/Essigester = 7:3)
Massenspektrum (ESI⁺): m/z = 169 [M+H]⁺

### Herstellung der Endverbindungen

### Beispiel 1

### 1-[(4-Phenylamino-chinazolin-2-yl)methyl]-3-methyl-7-(2-butin-1-yl)-8-(3-aminopiperidin-1-yl)-xanthin

Ein Gemisch aus 400 mg 1-[(4-Phenylamino-chinazolin-2-yl)methyl]-3-methyl-7-(2-butin-1-yl)-8-[3-(tert.-butyloxycarbonylamino)-piperidin-1-yl]-xanthin in 10 ml Methylenchlorid wir mit 2 ml isopropanolischer Salzsäure (5-6 M) versetzt und drei Stunden bei Raumtemperatur gerührt. Anschließend wird das Reaktionsgemisch mit Methylenchlorid verdünnt, mit Eiswasser versetzt und mit 3 M Kaliumcarbonatlösung alkalisch gestellt. Die wässrige Phase wird mit Methylenchlorid extrahiert. Die vereinigten Extrakte werden mit Wasser gewaschen, über Magnesiumsulfat getrocknet und eingeengt. Der Kolbenrückstand wird mit Diethylether verrührt, abgesaugt, mit Diethylether nachgewaschen und im Vakuum getrocknet.
Ausbeute: 274 mg (81 % der Theorie)
R_{f}-Wert: 0.50 (Kieselgel, Methylenchlorid/Methano)konz. wässriges Ammoniak = 90:10:0.1)
Massenspektrum (ESI⁺): m/z = 550 [M+H]⁺
Analog Beispiel 1 werden folgende Verbindungen erhalten:
(1) 1-[(4-Benzylamino-chinazolin-2-yl)methyl]-3-methyl-7-(2-butin-1-yl)-8-(3-aminopiperidin-1-yl)-xanthin
   R_{f}-Wert: 0.43 (Kieselgel, Methylenchlorid/Methanol/konz. wässriges Ammoniak = 90:10:0.1)
   Massenspektrum (ESI⁺): m/z = 564 [M+H]⁺
(2) 1-[(2-Methyl-chinolin-4-yl)methyl]-3-methyl-7-(2-butin-1-yl)-8-((*R*)-3-aminopiperidin-1-yl)-xanthin
   Massenspektrum (ESI⁺): m/z = 472 [M+H]⁺
(3) 1-[(3-Cyano-naphthalin-1-yl)methyl]-3-methyl-7-(2-butin-1-yl)-8-((*R*)-3-aminopiperidin-1-yl)-xanthin
   R_{f}-Wert: 0.55 (Kieselgel, Methylenchlorid/Methanollkonz. wässriges Ammoniak = 90:10:1)
   Massenspektrum (ESI⁺): m/z = 482 [M+H]⁺
(4) 1-[(2-Phenyl-chinazolin-4-yl)methyl]-3-methyl-7-(2-butin-1-yl)-8-((*R*)-3-aminopiperidin-1-yl)-xanthin
   R_{f}-Wert: 0.45 (Reversed Phase DC-Fertigplatte (E. Merck), Acetonitril/Wasser/Trifluoressigsäure = 50:50:1)
   Massenspektrum (ESI⁺): m/z = 535 [M+H]⁺
(5) 1-[(4-Cyano-isochinolin-1-yl)methyl]-3-methyl-7-(2-butin-1-yl)-8-((*R*)-3-aminopiperidin-1-yl)-xanthin
   R_{f}-Wert: 0.15 (Kieselgel, Methylenchlorid/Methanol/konz. wässriges Ammoniak = 90:10:1)
   Massenspektrum (ESI⁺): m/z = 483 [M+H]⁺
(6) 1-[(4-Cyano-chinolin-2-yl)methyl]-3-methyl-7-(2-butin-1-yl)-8-((*R*)-3-aminopiperidin-1-yl)-xanthin
   Massenspektrum (ESI⁺): m/z = 483 [M+H]⁺
(7) 1-[2-(3-Cyclopropyloxy-phenyl)-2-oxo-ethyl]-3-methyl-7-(2-butin-1-yl)-8-((*R*)-3-amino-piperidin-1-yl)-xanthin
   R_{f}-Wert: 0.45 (Reversed Phase DC-Fertigplatte (E. Merck), Acetonitril/Wasser/Trifluoressigsäure = 50:50:1)
   Massenspektrum (ESI⁺): m/z = 491 [M+H]⁺
(8) 1-[2-(3-Cyclopropylmethoxy-phenyl)-2-oxo-ethyl]-3-methyl-7-(2-butin-1-yl)-8-((*R*)-3-amino-piperidin-1-yl)-xanthin
   R_{f}-Wert- 0.35 (Reversed Phase DC-Fertigplatte (E. Merck), Acetonitril/Wasser/
   Trifluoressigsäure = 50:50:1)
   Massenspektrum (ESI⁺): m/z = 505 [M+H]⁺
(9) 1-[2-(3-Cyclobutyloxy-phenyl)-2-oxo-ethyl]-3-methyl-7-(2-butin-1-yl)-8-((*R*)-3-amino-piperidin-1-yl)-xanthin
   R_{f}-Wert: 0.40 (Reversed Phase DC-Fertigplatte (E. Merck), Acetonitril/Wasser/Trifluoressigsäure = 50:50:1)
   Massenspektrum (ESI⁺) m/z = 505 [M+H]⁺
(10) 1-[(1-Cyano-isochinolin-3-yl)methyl]-3-methyl-7-(2-butin-1-yl)-8-((*R*)-3-aminopiperidin-1-yl)-xanthin
   Massenspektrum (ESI⁺): m/z = 483 [M+H]⁺
(11) 1-[(2,4-Methoxy-naphthalin-1-yl)methyl]-3-methyl-7-(2-butin-1-yl)-8-((*R*)-3-aminopiperidin-1-yl)-xanthin
   R_{f}-Wert: 0.55 (Kieselgel, Methylenchlorid/Methanol/konz. wässriges Ammoniak = 90:10:0.1)
   Massenspektrum (ESI⁺): m/z = 517 [M+H]⁺
(12) 1-[(2,3-Dimethyl-chinoxalin-6-yl)methyl]-3-methyl-7-(2-butin-1-yl)-8-((*R*)-3-aminopiperidin-1-yl)-xanthin
   R_{f}-Wert: 0.48 (Kieselgel, Methylenchlorid/Methanol/konz. wässriges Ammoniak = 90:10:0.1)
   Massenspektrum (ESI⁺): m/z = 487 [M+H]⁺
(13) 1-[(6-Amino-chinolin-2-yl)methyl]-3-methyl-7-(2-butin-1-yl)-8-((*R*)-3-aminopiperidin-1-yl)-xanthin
   R_{f}-Wert: 0.40 (Kieselgel, Methylenchlorid/Methanol/konz. wässeriges Ammoniak = 90:10:0.1)
   Massenspektrum (ESI⁺): m/z = 473 [M+H]⁺
(14) 1-[(Chinoxalin-5-yl)methyl]-3-methyl-7-(2-butin-1-yl)-8-((*R*)-3-amino-piperidin-1-yl)-xanthin-hydrochlorid
   Massenspektrum (ESI⁺): m/z = 459 [M+H]⁺
(15) 1-[(6-Methoxy-chinolin-2-yl)methyl]-3-methyl-7-(2-butin-1-yl)-8-((*R*)-3-aminopiperidin-1-yl)-xanthin
   R_{f}-Wert: 0.50 (Kieselgel, Methylenchlorid/Methanol/konz. wässriges Ammoniak = 90:10:0.1)
   Massenspektrum (ESI⁺): m/z 488 [M+H]⁺
(16) 1-[(6-Phenyl-pyridin-2-yl)methyl]-3-methyl-7-(2-butin-1-yl)-8-((*R*)-3-aminopiperidin-1-yl)-xanthin
   R_{f}-Wert: 0.37 (Kieselgel, Methylenchlorid/Methanol/konz. wässriges Ammoniak = 90:10:1)
   Massenspektrum (ESI⁺): m/z = 484 [M+H]⁺
(17) 1-{[(4-(Pyridin-2-yl)-isochinolin-1-yl]methyl}-3-methyl-7-(2-butin-1-yl)-8-((*R*)-3-amino-piperidin-1-yl)-xanthin
   R_{f}-Wert: 0.37 (Kieselgel, Methylenchlorid/Methanol/konz. wässriges Ammoniak = 80:20:1)
   Massenspektrum (ESI⁺): m/z = 535 [M+H]⁺
(18) 1-[(7-Fluor-chinolin-2-yl)methyl]-3-methyl-7-(2-butin-1-yl)-8-((*R*)-3-aminopiperidin-1-yl)-xanthin
   R_{f}-Wert: 0.58 (Kieselgel, Methylenchlorid/Methanol/konz. wässriges Ammoniak = 90:10:1)
   Massenspektrum (ESI⁺): m/z = 476 [M+H]⁺
(19) 1-[(8-Amino-chinolin-2-yl)methyl]-3-methyl-7-(2-butin-1-yl)-8-((*R*)-3-aminopiperidin-1-yf)-xanthin
   R_{f}-Wert: 0.50 (Kieselgel, Methylenchlorid/Methanol/konz. wässriges Ammoniak = 90:10:1)
   Massenspektrum (ESI⁺): m/z = 473 [M+H]⁺
(20) 1-[(6-Fluor-chinolin-2-yl)methyl]-3-methyl-7-(2-butin-1-yl)-8-((R)-3-aminopiperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺) : m/z = 476 [M+H]⁺
(21) 1-{2-Oxo-2-[2-(pyridin-3-yl)-phenyl]-ethyl}-3-methyl-7-(2-butin-1-yl)-8-((*R*)-3-amino-piperidin-1-yl)-xanthin
   R_{f}-Wert: 0.55 (Reversed Phase DC-Fertigplatte (E. Merck), Acetonitril/Wasser/Trifluoressigsäure = 50:50:1)
   Massenspektrum (ESI⁺): m/z = 512 [M+H]⁺
(22) 1-[(4-Ethyl-chinazolin-2-yl)methyl]-3-methyl-7-(2-butin-1-yl)-8-((*R*)-3-aminopiperidin-1-yl)-xanthin
   R_{f}-Wert: 0.45 (Kieselgel, Methylenchlorid/Methanol/konz. wässriges Ammoniak = 90:10:0.1)
   Massenspektrum (ESI⁺): m/z = 487 [M+H]⁺
(23) 1-[(4-Cyclopropyl-chinazolin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 499 [M+H]⁺
(24) 1-[(4-Methoxy-chinolin-2-yl)methyl]-3-methyl-7-(2-butin-1-yl)-8-((*R*)-3-aminopiperidin-1-yl)-xanthin
   Massenspektrum (ESI⁺): m/z = 488 [M+H]⁺
(25) 1-[(2-Phenyl-pyrimidin-4-yl)methyl]-3-methyl-7-(2-butin-1-yl)-8-((*R*)-3-aminopiperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 485 [M+H]⁺
(26) 1-[([1,5]Naphthyridin-3-yl)methyl]-3-methyl-7-(2-butin-1-yl)-8-((*R*)-3-aminopiperidin-1-yl)-xanthin
   R_{f}-Wert: 0.52 (Kieselgel, Methylenchlorid/Methanol/konz. wässriges Ammoniak = 90:10:1)
   Massenspektrum (ESI⁺): m/z = 459 [M+H]⁺
(27) 1-[(3-Cyano-4-methyl-chinolin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.50 (Reversed Phase DC-Fertigplatte (E. Merck), Acetonitril/Wasser/Trifluoressigsäure = 50:50:1)
   Massenspektrum (ESI⁺): m/z = 497 [M+H]⁺
(28) 1-[(4,5-Dimethyl-chinazolin-2-yl)methyl]-3-methyl-7-(2-butin-1-yl)-8-((*R*)-3-aminopiperidin-1-yl)-xanthin
   (BOC-Spaltung erfolgt mit Trifluoressigsäure)
   Massenspektrum (ESI⁺): m/z = 487 [M+H]⁺
(29) 1-[(5-Cyano-chinolin-2-yl)methyl]-3-methyl-7-(2-butin-1-yl)-8-((*R*)-3-aminopiperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 483 [M+H]⁺
(30) 1-[(3-Cyano-chinolin-2-yl)methyl]-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.50 (Reversed Phase DC-Fertigplatte (E. Merck), Acetonitril/Wasser/Trifluoressigsäure = 50:50:1)
   Massenspektrum (ESI⁺): m/z = 483 [M+H]⁺
(31) 1-[(4-Phenyl-pyrimidin-2-yl)methyl]-3-methyl-7-(2-butin-1-yl)-8-((*R*)-3-aminopiperidin-1-yl)-xanthin
   R_{f}-Wert: 0.50 (Kieselgel, Methylenchlorid/methanol/konz. wässriges Ammoniak = 90:10:1)
   Massenspektrum (ESI⁺): m/z = 485 [M+H]⁺
(32) 1-[(2-Methyl-chinazolin-4-yl)methyl]-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.38 (Kieselgel, Methylenchlorid/Methanol/konz. wässriges Ammoniak = 90:10:1)
   Massenspektrum (ESI⁺): m/z = 473 [M+H]⁺
(33) 1-[(1,4-Dicyano-naphthalin-2-yl)methyl]-3-methyl-7-(2-butin-1-yl)-8-((*R*)-3-aminopiperidin-1-yl)-xanthin-hydrochlorid
   R_{f}-Wert: 0.86 (Kieselgel, Methylenchlorid/Methanol/konz. wässriges Ammoniak = 90:10:1)
   Massenspektrum (ESI⁺): m/z = 507 [M+H]⁺
(34) 1-{2-Oxo-2-[2-(pyridin-4-yl)-phenyl]-ethyl}-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.55 (Reversed Phase DC-Fertigplatte (E. Merck), Acetonitril/Wasser/Trifluoressigsäure = 50:50:1)
   Massenspektrum (ESI⁺): m/z = 512 [M+H]⁺
(35) 1-[(6,7-Dimethoxy-chinolin-2-yl)methyl]-3-methyl-7-(2-butin-1-yl)-8-((*R*)-3-aminopiperidin-1-yl)-xanthin
   R_{f}-Wert: 0.45 (Kieselgel, Methylenchlorid/Methanol/konz. wässriges Ammoniak = 90:10:1)
   Massenspektrum (ESI⁺): m/z = 518 [M+H]⁺
(36) 1-[(Chinazolin-6-yl)methyl]-3-methyl-7-(2-butin-1-yl)-8-((*R*)-3-amino-piperidin-1-yl)-xanthin-hydrochlorid
   R_{f}-Wert: 0.50 (Kieselgel, Methylenchlorid/Methanol/konz. wässriges Ammoniak = 90:10:0.1)
   Massenspektrum (ESI⁺): m/z = 459 [M+H]⁺
(37) 1-[(4-Cyano-chinazolin-2-yl)methyl]-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.50 (Kieselgel, Methylenchlorid/Methanol/konz. wässriges Ammoniak = 90:10:1)
   Massenspektrum (ESI⁺): m/z = 484 [M+H]⁺
(38) 1-[(Chinazolin-7-yl)methyl]-3-methyl-7-(2-butin-1-yl)-8-((*R*)-3-amino-piperidin-1-yl)-xanthin
   R_{f}-Wert: 0.43 (Kieselgel, Methylenchlorid/Methanol/konz. wässriges Ammoniak = 90:10:0.1)
   Massenspektrum (ESI⁺): m/z = 459 [M+H]⁺
(39) 1-(2-Cyano-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.35 (Kieselgel, Methylenchlorid/Methanol/konz. wässriges Ammoniak = 90:10:1)
   Massenspektrum (ESI⁺): m/z = 432 [M+H]⁺
(40) 1-(3-Cyano-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.40 (Kieselgel, Methylenchlorid/Methanol/konz. wässriges Ammoniak = 90:10:1)
   Massenspektrum (ESI⁺): m/z = 432 [M+H]⁺
(41) 1-(4-Cyano-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.31 (Kieselgel, Methylenchlorid/Methanol/konz. wässriges Ammoniak = 90:10:1)
   Massenspektrum (ESI⁺): m/z = 432 [M+H]⁺
(42) 1-[(Pyridin-2-yl)methyl]-3-methyl-7-(2-butin-1-yl)-8-((*R*)-3-amino-piperidin-1-yl)-xanthin
   R_{f}-Wert: 0.52 (Kieselgel, Methylenchlorid/Methanol/konz. wässriges Ammoniak = 90:10:1)
   Massenspektrum (ESI⁺): m/z = 408 [M+H]⁺
(43) 1-Benzyl-3-methyl-7-(2-butin-1-yl)-8-((*R*)-3-amino-piperidin-1-yl)-xanthin (BOC-Spaltung erfolgt mit Trifluoressigsäure)
   Schmelzpunkt: 207-209°C
   Massenspektrum (ESI⁺): m/z = 407 [M+H]⁺
(44) 1-(4-Methoxy-benzyl)-3-methyl-7-(2-butin-1-yl)-8-((*R*)-3-amino-piperidin-1-yl)-xanthin-hydrochlorid
   R_{f}-Wert: 0.50 (Kieselgel, Methylenchlorid/Methanol/konz. wässriges Ammoniak = 90:10:0.1)
   Massenspektrum (ESI⁺): m/z = 437 [M+H]⁺
(45) 1-(2-Chlor-benzyl)-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:0.1)
   Massenspektrum (ESI⁺): m/z = 441, 443 [M+H]⁺
(46) 1-(2,6-Dicyano-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 457 [M+H]⁺
(47) 1-(2-Cyano-4-brom-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 510, 512 [M+H]⁺
(48) 1-(3-Fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 425 [M+H]⁺
(49) 1-(3,5-Dimethoxy-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 467 [M+H]⁺
(50) 1-(2-Fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 425 [M+H]⁺
(51) 1-[(6-Cyano-pyridin-2-yl)methyl]-3-methyl-7-(2-butin-1-yl)-8-((*R*)-3-aminopiperidin-1-yl)-xanthin
   (BOC-Spaltung erfolgt mit Trifluoressigsäure)
   Massenspektrum (ESI⁺): m/z = 433 [M+H]⁺
(52) 1-[(3-Cyano-pyridin-2-yl)methyl]-3-methyl-7-(2-butin-1-yl)-8-((*R*)-3-aminopiperidin-1-yl)-xanthin
   (BOC-Spaltung erfolgt mit Trifluoressigsäure)
   Massenspektrum (ESI⁺): m/z = 433 [M+H]⁺
(53) 1-(2-Cyano-3-chlor-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 466, 468 [M+H]⁺
(54) 1-(4-Fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 425 [M+H]⁺
(55) 1-(4-Chlor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 441, 443 [M+H]⁺
(56) 1-(2-Cyano-4-fluor-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 450 [M+H]⁺
(57) 1-(3-Cyano-4-fluor-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 450 [M+H]⁺
(58) 1-(2-Chlor-4-cyano-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 466, 468 [M+H]⁺
(59) 1-[(5-Methoxycarbonyl-furan-2-yl)methyl]-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 455 [M+H]⁺
(60) 1-(2-Trifluormethyl-4-cyano-benzyl)-3-methyl-7-(2-butin-1-yl)-8-((*R*)-3-aminopiperidin-1-yl)-xanthinx x Trifluoressigsäure
   (BOC-Spaltung erfolgt mit Trifluoressigsäure)
   Massenspektrum (ESI⁺): m/z = 500 [M+H]⁺
(61) 1-(3,5-Dicyano-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 457 [M+H]⁺
(62) 1-(3-Nitro-4-cyano-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 477 [M+H]⁺
(63) 1-[(2-Cyano-pyridin-3-yl)methyl]-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.50 (Reversed Phase DC-Fertigplatte (E. Merck), Acetonitril/Wasser/Trifluoressigsäure = 50:50:1)
   Massenspektrum (ESI⁺): m/z = 433 [M+H]⁺
(64) 1-(2-Cyano-4-methoxy-benzyl)-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.50 (Reversed Phase DC-Fertigplatte (E. Merck), Acetonitril/Wasser/Trifluoressigsäure = 50:50:1)
   Massenspektrum (ESI⁺): m/z = 462 [M+H]⁺
(65) 1-(2-Cyano-5-methoxy-benzyl)-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.45 (Reversed Phase DC-Fertigplatte (E. Merck), Acetonitril/Wasser/Trifluoressigsäure = 50:50:1)
   Massenspektrum (ESI⁺): m/z = 462 [M+H]⁺
(66) 1-(3-Methoxy-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 437 [M+H]⁺
(67) 1-(3-Trifluormethyl-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.60 (Kieselgel, Methylenchlorid/Methanol/konz. wässriges Ammoniak = 90:10:0.1)
   Massenspektrum (ESI⁺): m/z = 475 [M+H]⁺
(68) 1-(3,4-Dimethoxy-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.50 (Kieselgel, Methylenchlorid/Methanol/konz. wässriges Ammoniak = 90:10:0.1)
   Massenspektrum (ESI⁺): m/z = 467 [M+H]⁺
(69) 1-(3-Chlor-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 441, 443 [M+H]⁺
(70) 1-(4-Trifluormethyl-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 475 [M+H]⁺
(71) 1-[([2,2']Bipyridinyl-6-yl)methyl-3-methyl-7-(2-butin-1-yl)-8-((*R*)-3-aminopiperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 485 [M+H]⁺
(72) 1-(3,4-Dimethoxy-6-fluor-benzyl)-3-methyl-7-(2-butin-1-yl)-8-((*R*)-3-aminopiperidin-1-yl)-xanthin
   R_{f}-Wert: 0.50 (Kieselgel, Methylenchlorid/Methanol/konz. wässriges Ammoniak = 90:10:0.1)
   Massenspektrum (ESI⁺): m/z = 485 [M+H]⁺
(73) 1-[(6-Fluor-pyridin-2-yl)methyl]-3-methyl-7-(2-butin-1-yl)-8-((*R*)-3-aminopiperidin-1-yl)-xanthin
   R_{f}-Wert: 0.41 (Kieselgel, Methylenchlorid/Methanol/konz. wässriges Ammoniak = 90:10:1)
   Massenspektrum (ESI⁺): m/z = 426 [M+H]⁺
(74) 1-[(5-Cyano-6-methoxy-pyridin-2-yl)methyl]-3-methyl-7-(2-butin-1-yl)-8-((*R*)-3-amino-piperidin-1-yl)-xanthin
   R_{f}-Wert: 0.40 (Kieselgel, Methylenchlorid/Methanol/konz. wässriges Ammoniak = 90:10:1)
   Massenspektrum (ESI⁺): m/z = 463 [M+H]⁺
(75) 1-(2,6-Difluor-benzyl)-3-methyl-7-(2-butin-1-yl)-8-((*R*)-3-amino-piperidin-1-yl)-xanthin
   R_{f}-Wert: 0.41 (Kieselgel, Methylenchlorid/Methanol/konz. wässriges Ammoniak = 90:10:1)
   Massenspektrum (ESI⁺): m/z = 443 [M+H]⁺
(76) 1-(3-Trifluormethoxy-benzy))-3-methyl-7-(2-butin-1-yl)-8-((*R*)-3-amino-piperidin-1-yl)-xanthin
   R_{f}-Wert: 0.36 (Kieselgel, Methylenchlorid/Methanol/konz. wässriges Ammoniak = 90:10:1)
   Massenspektrum (ESI⁺): m/z = 491 [M+H]⁺
(77) 1-(4-Trifluormethoxy-benzyl)-3-methyl-7-(2-butin-1-yl)-8-((*R*)-3-amino-piperidin-1-yl)-xanthin
   R_{f}-Wert: 0.38 (Kieselgel, Methylenchlorid/Methanol/konz. wässriges Ammoniak = 90:10:1)
   Massenspektrum (ESI⁺): m/z = 491 [M+H]⁺
(78) 1-[(2-Cyano-pyridin-4-yl)methyl]-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.60 (Reversed Phase DC-Fertigplatte (E. Merck), Acetonitril/Wasser/Trifluoressigsäure = 50:50:1)
   Massenspektrum (ESI⁺): m/z = 433 [M+H]⁺
(79) 1-[(5-Cyano-pyridin-2-yl)methyl]-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.60 (Reversed Phase DC-Fertigplatte (E. Merck), Acetonitril/Wasser/Trifluoressigsäure = 50:50:1)
   Massenspektrum (ESI⁺): m/z = 433 [M+H]⁺
(80) 1-[(Pyrimidin-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.60 (Reversed Phase DC-Fertigplatte (E. Merck), Acetonitril/Wasser/ Trifluoressigsäure = 50:50:1)
   Massenspektrum (ESI⁺): m/z = 409 [M+H]⁺
(81) 1-[(4-Methyl-pyrimidin-2-yl)methyl]-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.65 (Reversed Phase DC-Fertigplatte (E. Merck), Acetonitril/Wasser/Trifluoressigsäure = 50:50:1)
   Massenspektrum (ESI⁺): m/z = 423 [M+H]⁺
(82) 1-[(4,6-Dimethyl-pyrimidin-2-yl)methyl]-3-methyl-7-(2-butin-1-yl)-8-((*R*)-3-aminopiperidin-1-yl)-xanthin
   (BOC-Spaltung erfolgt mit Trifluoressigsäure)
   Schmelzpunkt: 202-204°C
   Massenspektrum (ESI⁺): m/z = 437 [M+H]⁺
(83) 1-[(Chinoxalin-6-yl)methyl]-3-methyl-7-(2-butin-1-yl)-8-((*R*)-3-amino-piperidin-1-yl)-xanthin
   R_{f}-Wert: 0.50 (Kieselgel, Methylenchlorid/Methanol/konz. wässriges Ammoniak = 90:10:0.1)
   Massenspektrum (ESI⁺): m/z = 459 [M+H]⁺
(84) 1-(3-Fluor-4-methoxy-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 455 [M+H]⁺
(85) 1-(3,4-Difluor-benzyl)-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.45 (Kieselgel, Methylenchlorid/Methanol/konz. wässriges Ammoniak = 90:10:0.1)
   Massenspektrum (ESI⁺): m/z = 443 [M+H]⁺
(86) 1-(2-Fluor-5-methoxy-benzyl)-3-methyl-7-(2-butin-1-yl)-8-((*R*)-3-amino-piperidin-1-yl)-xanthin
   R_{f}-Wert: 0.39 (Kieselgel, Methytenchtorid/Methanol/konz. wässriges Ammoniak = 90:10:1)
   Massenspektrum (ESI⁺): m/z = 455 [M+H]⁺
(87) 1-(2-Fluor-3-methoxy-benzyl)-3-methyl-7-(2-butin-1-yl)-8-((*R*)-3-amino-piperidin-1-yl)-xanthin
   R_{f}-Wert: 0.41 (Kieselgel, Methylenchlorid/Methanol/konz. wässriges Ammoniak = 90:10:1)
   Massenspektrum (ESI⁺): m/z = 455 [M+H]⁺
(88) 1-[(4-Cyano-isochinolin-3-yl)methyl]-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.40 (Kieselgel, Methylenchlorid/Methanol/konz. wässriges Ammoniak = 90:10:0.1)
   Massenspektrum (ESI⁺): m/z = 483 [M+H]⁺
(89) 1-(2-Fluor-4-methoxy-benzyl)-3-methyl-7-(2-butin-1-yl)-8-((*R*)-3-amino-piperidin-1-yl)-xanthin
   R_{f}-Wert: 0.45 (Kieselgel, Methylenchlorid/Methanol/konz. wässriges Ammoniak = 90:10:1)
   Massenspektrum (ESI⁺): m/z = 455 [M+H]⁺
(90) 1-[(Furan-2-yl)methyl]-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 397 [M+H]⁺
(91) 1-(3,4-Dicyano-benzyl)-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 457 [M+H]⁺
(92) 1-[(Furan-3-yl)methyl]-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 397 [M+H]⁺
(93) 1-[(5-Methyl-furan-2-yl)methyl]-3-methyl-7-(2-butin-1-yl)-8-((*R*)-3-aminopiperidin-1-yl)-xanthin
   (BOC-Spaltung erfolgt mit Trifluoressigsäure)
   Massenspektrum (ESI⁺): m/z = 411 [M+H]⁺
(94) 1-[(5-Brom-furan-2-yl)methyl]-3-methyl-7-(2-butin-1-yl)-8-((*R*)-3-amino-piperidin-1-yl)-xanthin x Trifluoressigsäure
   (BOC-Spaltung erfolgt mit Trifluoressigsäure)
   Massenspektrum (ESI⁺): m/z = 475, 477 [M+H]⁺
(95) 1-(4-Cyano-2-fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 450 [M+H]⁺
(96) 1-(2-Cyano-5-fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 450 [M+H]⁺
(97) 1-[(5-Cyano-furan-2-yl)methyl]-3-methyl-7-(2-butin-1-yl)-8-((*R*)-3-aminopiperidin-1-yl)-xanthin x Trifluoressigsäure
   (BOC-Spaltung erfolgt mit Trifluoressigsäure)
   Massenspektrum (ESI⁺): m/z = 422 [M+H]⁺
(98) 1-(2-Cyano-6-fluor-benzyl)-3-methyl-7-(2-butin-1-yf)-8-((*R*)-3-amino-piperidin-1-yl)-xanthin
   (BOC-Spaltung erfolgt mit Trifluoressigsäure)
   Massenspektrum (ESI⁺): m/z = 450 [M+H]⁺
(99) 1-(4-Cyano-3-fluor-benzyl)-3-methyl-7-(2-butin-1-yl)-8-((*R*)-3-amino-piperidin-1-yl)-xanthin
   (BOC-Spaltung erfolgt mit Trifluoressigsäure)
   Massenspektrum (ESI⁺): m/z = 450 [M+H]⁺
(100) 1-(2-Cyano-3-methoxy-benzyl)-3-methyl-7-(2-butin-1-yl)-8-((*R*)-3-aminopiperidin-1-yl)-xanthin
   R_{f}-Wert: 0.50 (Kieselgel, Methytenchtorid/Methanol/konz. wässriges Ammoniak = 90:10:0.1)
   Massenspektrum (ESI⁺): m/z = 462 [M+H]⁺
(101) 1-[(8-Cyano-chinolin-7-yl)methyl]-3-methyl-7-(2-butin-1-yl)-8-((*R*)-3-aminopiperidin-1-yl)-xanthin
   (BOC-Spaltung erfolgt mit Trifluoressigsäure)
   Massenspektrum (ESI⁺): m/z = 483 [M+H]⁺
(102) 1-[(4-Cyano-pyridin-3-yl)methyl]-3-methyl-7-(2-butin-1-yl)-8-((*R*)-3-aminopiperidin-1-yl)-xanthin
   (BOC-Spaltung erfolgt mit Trifluoressigsäure)
   Schmelzpunkt: 166°C
   Massenspektrum (ESI⁺): m/z = 433 [M+H]⁺
(103) 1-[(8-Cyano-chinolin-2-yl)methyl]-3-methyl-7-(2-butin-1-yl)-8-((*R*)-3-aminopiperidin-1-yl)-xanthin
   (BOC-Spaltung erfolgt mit Trifluoressigsäure)
   R_{f}-Wert: 0.50 (Kieselgel, Methylenchlorid/Methanol/konz. wässriges Ammoniak = 90:10:0.1)
   Massenspektrum (ESI⁺): m/z = 483 [M+H]⁺
(104) 1-[(1-Methyl-1 H-benzotriazol-5-yl)methyl]-3-methyl-7-(2-butin-1-yl)-8-((*R*)-3-amino-piperidin-1-yl)-xanthin
   (BOC-Spaltung erfolgt mit Trifluoressigsäure)
   R_{f}-Wert: 0.60 (Reversed Phase DC-Fertigplatte (E. Merck), Acetonitril/Wasser/Trifluoressigsäure = 50:50:1)
   Massenspektrum (ESI⁺): m/z = 462 [M+H]⁺
(105) 1-[(3-Cyano-pyridin-4-yl)methyl]-3-methyl-7-(2-butin-1-yl)-8-((*R*)-3-aminopiperidin-1-yl)-xanthin x Trifluoressigsäure
   (BOC-Spaltung erfolgt mit Trifluoressigsäure)
   R_{f}-Wert: 0.65 (Reversed Phase DC-Fertigplatte (E. Merck), Acetonitril/Wasser/Trifluoressigsäure = 50:50:1)
   Massenspektrum (ESI⁺): m/z = 433 [M+H]⁺
(106) 1-[(3-Cyano-pyridin-2-yl)methyl]-3-methyl-7-(2-butin-1-yl)-8-((*S*)-3-aminopiperidin-1-yl)-xanthin
   (BOC-Spaltung erfolgt mit Trifluoressigsäure)
   R_{f}-Wert: 0.60 (Reversed Phase DC-Fertigplatte (E. Merck), Acetonitril/Wasser/Trifluoressigsäure = 50:50:1)
   Massenspektrum (ESI⁺): m/z = 433 [M+H]⁺
(107) 1-[(4-Cyano-benzo[1,3]dioxol-5-yl)methyl]-3-methyl-7-(2-butin-1-yl)-8-((*R*)-3-amino-piperidin-1-yl)-xanthin
   R_{f}-Wert: 0.45 (Kieselgel, Methylenchlorid/Methanol/konz. wässriges Ammoniak = 90:10:0.1)
   Massenspektrum (ESI⁺): m/z = 476 [M+H]⁺

Analog den vorstehenden Beispielen und anderen literaturbekannten Verfahren können auch folgende Verbindungen erhalten werden:
(1) 1-(2-Cyano-4-fluor-benzyl)-3-methyl-7-(2-butin-1-yl)-8-((R)-3-amino-piperidin-1-yl)-xanthin
(2) 1-(2-Cyano-5-fluor-benzyl)-3-methyl-7-(2-butin-1-yl)-8-((R)-3-amino-piperidin-1-yl)-xanthin
(3) 1-(2-Cyano-6-fluor-benzyl)-3-methyl-7-(2-butin-1-yl)-8-((R)-3-amino-piperidin-1-yl)-xanthin
(4) 1-(3-Cyano-4-fluor-benzyl)-3-methyl-7-(2-butin-1-yl)-8-((R)-3-amino-piperidin-1-yl)-xanthin
(5) 1-(3,5-Dicyano-benzyl)-3-methyl-7-(2-butin-1-yl)-8-((R)-3-amino-piperidin-1-yl)-xanthin
(6) 1-(3,4-Dicyano-benzyl)-3-methyl-7-(2-butin-1-yl)-8-((R)-3-amino-piperidin-1-yl)-xanthin
(7) 1-(3-Nitro-4-cyano-benzyl)-3-methyl-7-(2-butin-1-yl)-8-((R)-3-amino-piperidin-1-yl)-xanthin
(8) 1-(2-Chlor-4-cyano-benzyl)-3-methyl-7-(2-butin-1-yl)-8-((R)-3-amino-piperidin-1-yl)-xanthin
(9) 1-(2-Fluor-4-cyano-benzyl)-3-methyl-7-(2-butin-1-yl)-8-((R)-3-amino-piperidin-1-yl)-xanthin
(10) 1-(2-Trifluormethyl-4-cyano-benzyl)-3-methyl-7-(2-butin-1-yl)-8-((R)-3-aminopiperidin-1-yl)-xanthin
(11) 1-[(5-Cyano-pyridin-2-yl)methyl]-3-methyl-7-(2-butin-1-yl)-8-((R)-3-aminopiperidin-1-yl)-xanthin
(12) 1-[(4-Cyano-pyridin-2-yl)methyl]-3-methyl-7-(2-butin-1-yl)-8-((R)-3-aminopiperidin-1-yl)-xanthin
(13) 1-[(4-Cyano-pyridin-3-yl)methyl]-3-methyl-7-(2-butin-1-yl)-8-((R)-3-aminopiperidin-1-yl)-xanthin
(14) 1-[(3-Cyano-pyridin-4-yl)methyl]-3-methyl-7-(2-butin-1-yl)-8-((R)-3-aminopiperidin-1-yl)-xanthin
(15) 1-[(2-Cyano-pyridin-3-yl)methyl]-3-methyl-7-(2-butin-1-yl)-8-((R)-3-aminopiperidin-1-yl)-xanthin
(16) 1-1[(2-Cyano-pyridin-4-yl)methyl]-3-methyl-7-(2-butin-1-yl)-8-((R)-3-aminopiperidin-1-yl)-xanthin
(17) 1-[(5-Cyano-pyridin-3-yl)methyl]-3-methyl-7-(2-butin-1-yl)-8-((R)-3-aminopiperidin-1-yl)-xanthin
(18) 1-[(6-Cyano-pyridin-3-yl)methyl]-3-methyl-7-(2-butin-1-yl)-8-((R)-3-aminopiperidin-1-yl)-xanthin
(19) 1-(2-Cyano-4-methoxy-benzyi)-3-methyl-7-(2-butin-1-yl)-8-((R)-3-aminopiperidin-1-yl)-xanthin
(20) 1-(2-Cyano-5-methoxy-benzyl)-3-methyl-7-(2-butin-1-yl)-8-((R)-3-aminopiperidin-1-yl)-xanthin
(21) 1-[([2,2']Bipyridinyl-6-yl)methyl]-3-methyl-7-(2-butin-1-yl)-8-((R)-3-aminopiperidin-1-yl)-xanthin
(22) 1-[(5-Methoxy-pyridin-2-yl)methyl]-3-methyl-7-(2-butin-1-yl)-8-((R)-3-aminopiperidin-1-yl)-xanthin
(23) 1-[(6-Fluor-pyridin-2-yl)methyl]-3-methyl-7-(2-butin-1-yl)-8-((R)-3-aminopiperidin-1-yl)-xanthin
(24) 1-[(5-Cyano-6-methoxy-pyridin-2-yl)methyl]-3-methyl-7-(2-butin-1-yl)-8-((R)-3-amino-piperidin-1-yl)-xanthin
(25) 1-(2-Methoxy-benzyl)-3-methyl-7-(2-butin-1-yl)-8-((R)-3-amino-piperidin-1-yl)-xanthin
(26) 1-(3-Methoxy-benzyl)-3-methyl-7-(2-butin-1-yl)-8-((R)-3-amino-piperidin-1-yl)-xanthin
(27) 1-(3-Chlor-benzyl)-3-methyl-7-(2-butin-1-yl)-8-((R)-3-amino-piperidin-1-yl)-xanthin
(28) 1-(4-Trifluormethyl-benzyl)-3-methyl-7-(2-butin-1-yl)-8-((R)-3-amino-piperidin-1-yl)-xanthin
(29) 1-(3-Trifluormethyl-benzyl)-3-methyl-7-(2-butin-1-yl)-8-((R)-3-amino-piperidin-1-yl)-xanthin
(30) 1-(2-Trifluormethyl-benzyl)-3-methyl-7-(2-butin-1-yl)-8-((R)-3-amino-piperidin-1-yl)-xanthin
(31) 1-(3,4-Dimethoxy-benzyl)-3-methyl-7-(2-butin-1-yl)-8-((R)-3-amino-piperidin-1-yl)-xanthin
(32) 1-(3,4-Dimethoxy-6-fluor-benzyl)-3-methyl-7-(2-butin-1-yl)-8-((R)-3-aminopiperidin-1-yl)-xanthin
(33) 1-[(Benzo[1,3]dioxol-5-yl)methyl]-3-methyl-7-(2-butin-1-yl)-8-((R)-3-aminopiperidin-1-yl)-xanthin

### Beispiel 2

### Dragées mit 75 mg Wirksubstanz

| 1 | Dragéekern enthält: | |
|---|---|---|
| | Wirksubstanz | 75,0 mg |
| | Calciumphosphat | 93,0 mg |
| | Maisstärke | 35,5 mg |
| | Polyvinylpyrrolidon | 10,0 mg |
| | Hydroxypropylmethylcellulose | 15,0 mg |
| | Magnesiumstearat | 1,5 mg |
| | | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu

Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.

Dragéegewicht: 245 mg.

### Beispiel 3

Tabletten mit 100 mg Wirksubstanz

### Zusammensetzung:

| 1 | Tablette enthält: | |
|---|---|---|
| | Wirksubstanz | 100,0 mg |
| | Milchzucker | 80,0 mg |
| | Maisstärke | 34,0 mg |
| | Polyvinylpyrrolidon | 4,0 mg |
| | Magnesiumstearat | 2,0 mg |
| | | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 4

Tabletten mit 150 mg Wirksubstanz

### Zusammensetzung:

| 1 | Tablette enthält: | |
|---|---|---|
| | Wirksubstanz | 150,0 mg |
| | Milchzucker pulv. | 89,0 mg |
| | Maisstärke | 40,0 mg |
| | Kolloide Kieselgelsäure | 10,0 mg |
| | Polyvinylpyrrolidon | 10,0 mg |
| | Magnesiumstearat | 1,0 mg |
| | | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 5

### Hartgelatine-Kapseln mit 150 mg Wirksubstanz

| 1 | Kapsel enthält: | |
|---|---|---|
| | Wirkstoff | 150,0 mg |
| | Maisstärke getr. | ca. 180,0 mg |
| | Milchzucker pulv. | ca. 87,0 mg |
| | Magnesiumstearat | 3,0 mg |
| | | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.

| | |
|---|---|
| Kapselfüllung: ca. | 320 mg |
| Kapselhülle: | Hartgelatine-Kapsel Größe 1. |

### Beispiel 6

### Suppositorien mit 150 mg Wirksubstanz

| 1 | Zäpfchen enthält: | |
|---|---|---|
| | Wirkstoff | 150,0 mg |
| | Polyethylenglykol 1500 | 550,0 mg |
| | Polyethylenglykol 6000 | 460,0 mg |
| | Polyoxyethylensorbitanmonostearat | 840,0 mg |
| | | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 7

### Suspension mit 50 mg Wirksubstanz

| 100 ml Suspension enthalten: | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 8

### Ampullen mit 10 mg Wirksubstanz

### Zusammensetzung:

| | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 9

### Ampullen mit 50 mg Wirksubstanz

### Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 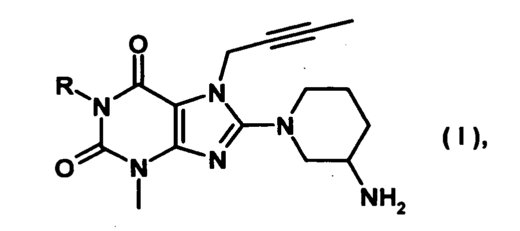 in der
R eine Benzyl-, 2-Fluorbenzyl-, 3-Fluorbenzyl-, 4-Fluorbenzyl-, 2,6-Difluor-benzyl-, 3,4-Difluor-benzyl-, 2-Chlorbenzyl-, 3-Chlorbenzyl- oder 4-Chlorbenzyl-Gruppe, eine 2- Trifluormethyl-benzyl-, 3-Trifluormethyl-benzyl- oder 4-Trifluormethyl-benzyl-Gruppe,
eine 3-Trifluormethoxy-benzyl- oder 4-Trifluormethoxy-benzyl-Gruppe,
eine 2-Cyanobenzyl-, 3-Cyanobenzyl- oder 4-Cyanobenzyl-Gruppe,
eine 2,6-Dicyanobenzyl-, 3,4-Dicyanobenzyl-, 3,5-Dicyanobenzyl-, 2-Trifluormethyl-4-cyano-benzyl-, 3-Nitro-4-cyano-benzyl-, 2-Cyano-3-methoxy-benzyl-, 2-Cyano-4-methoxy-benzyl-, 2-Cyano-5-methoxy-benzyl-, 2-Cyano-4-fluor-benzyl-, 2-Cyano-5-fluor-benzyl-, 2-Cyano-6-fluor-benzyl-, 3-Cyano-4-fluor-benzyl-, 4-Cyano-3-fluorbenzyl-, 2-Fluor-4-cyano-benzyl-, 2-Cyano-3-chlorbenzyl-, 2-Chlor-4-cyano-benzyl- oder 2-Cyano-4-brombenzyl-Gruppe,
eine 2-Methoxy-benzyl-, 3-Methoxy-benzyl-, 4-Methoxy-benzyl-, 2-Fluor-3-methoxybenzyl-, 2-Fluor-4-methoxy-benzyl-, 2-Fluor-5-methoxy-benzyl-, 3-Fluor-4-methoxybenzyl-, 3,4-Dimethoxy-benzyl-, 3,5-Dimethoxybenzyl- oder 3,4-Dimethoxy-6-fluorbenzyl-Gruppe,
eine (Benzo[1,3]dioxol-5-yl)methyl-Gruppe,
eine [(4-Cyano-benzo[1,3]dioxol-5-yl)methyl-Gruppe,
eine 2-(3-Cyclopropyloxy-phenyl)-2-oxo-ethyl-, 2-(3-Cyclopropylmethoxy-phenyl)-2-oxo-ethyl- oder 2-(3-Cyclobutyloxy-phenyl)-2-oxo-ethyl-Gruppe,
eine 2-Oxo-2-[2-(pyridin-3-yl)-phenyl]-ethyl- oder 2-Oxo-2-[2-(pyridin-4-yl)-phenyl]-ethyl-Gruppe,
eine (3-Cyano-naphthalin-1-yl)methyl-, (1,4-Dicyano-naphthalin-2-yl)methyl- oder (2,4-Dimethoxy-naphthalin-1-yl)methyl-Gruppe,
eine (Furan-2-yl)methyl-, (Furan-3-yl)methyl-, (5-Brom-furan-2-yl)methyl-, (5-Methylfuran-2-yl)methyl-, (5-Cyano-furan-2-yl)methyl- oder (5-Methoxycarbonyl-furan-2-yl)methyl-Gruppe,
eine (Pyridin-2-yl)methyl-, (6-Fluor-pyridin-2-yl)methyl- oder (5-Methoxy-pyridin-2-yl)methyl-Gruppe,
eine (3-Cyanopyridin-2₋yl)methyl-, (6-Cyanopyridin-2-yl)methyl-, (5-Cyano-pyridin-2-yl)methyl-, (4-Cyano-pyridin-2-yl)methyl-, (4-Cyano-pyridin-3-yl)methyl-, (3-Cyano-pyridin-4-yl)methyl-, (2-Cyano-pyridin-3-yl)methyl-, (2-Cyano-pyridin-4-yl)methyl-, (5-Cyano-pyridin-3-yl)methyl-, (6-Cyano-pyridin-3-yl)methyl- oder (5-Cyano-6-methoxy-pyridin-2-yl)methyl-Gruppe,
eine (6-Phenyl-pyridin-2-yl)methyl- oder eine ([2,2']Bipyridinyl-6-yl)methyl-Gruppe,
eine (Pyrimidin-2-yl)methyl-, (4-Methyl-pyrimidin-2-yl)methyl- oder (4,6-Dimethyl-pyrimidin-2-yl)methyl-Gruppe,
eine (2-Phenyl-pyrimidin-4-yl)methyl- oder (4-Phenyl-pyrimidin-2-yl)methyl-Gruppe, eine [(1-Methyl-1 H-benzotriazol-5-yl)methyl]-Gruppe,
eine (6-Fluor-chinolin-2-yl)methyl-, (7-Fluor-chinolin-2-yl)methyl-, (2-Methyl-chinolin-4-yl)methyl-, (3-Cyano-chinolin-2-yl)methyl-, (3-Cyano-4-methyl-chinolin-2-yl)methyl-, (4-Cyano-chinolin-2-yl)methyl-, (5-Cyano-chinolin-2-yl)methyl-, (8-Cyano-chinolin-2-yl)methyl-, (6-Amino-chinolin-2-yl)methyl-, (8-Amino-chinolin-2-yl)methyl-, (4-Methoxy-chinolin-2-yl)methyl-, (6-Methoxy-chinolin-2-yl)methyl-, (6,7-Dimethoxy-chinolin-2-yl)methyl- oder (8-Cyano-chinolin-7-yl)methyl-Gruppe,
eine (1-Cyano-isochinolin-3-yl)methyl-, (4-Cyano-isochinolin-1-yl)methyl-, (4-Cyano-isochinolin-3-yl)methyl- oder [(4-(Pyridin-2-yl)-isochinolin-1-yl]methyl-Gruppe,
eine (Chinazolin-6-yl)methyl-, (Chinazolin-7-yl)methyl-, (2-Methyl-chinazolin-4-yl)methyl-, (4,5-Dimethyl-chinazolin-2-yl)methyl-, (4-Ethyl-chinazolin-2-yl)methyl-, (4-Cyclopropyl-chinazolin-2-yl)methyl-, (2-Phenyl-chinazolin-4-yl)methyl-, (4-Cyano-chinazolin-2-yl)methyl-, (4-Phenylamino-chinazolin-2-yl)methyl- oder (4-Benzylamino-chinazolin-2-yl)methyl-Gruppe,
eine (Chinoxalin-5-yl)methyl-, (Chinoxalin-6-yl)methyl- oder (2,3-Dimethyl-chinoxalin-6-yl)methyl-Gruppe, oder
eine ([1,5]Naphthyridin-3-yl)methyl-Gruppe bedeutet,
deren Tautomere, Enantiomere, Diastereomere, deren Gemische und deren Salze.

2. Eine Verbindung der allgemeinen Formel 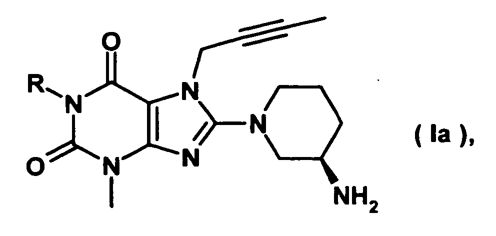 in der R wie in Anspruch 1 erwähnt definiert ist, sowie deren Tautomere und Salze.

3. Eine Verbindung der allgemeinen Formel 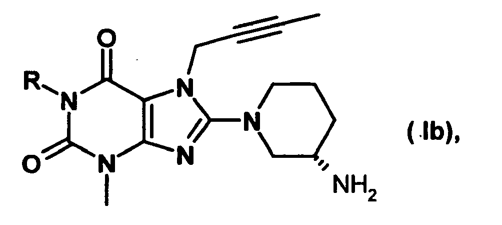 in der R wie in Anspruch 1 erwähnt definiert ist, sowie deren Tautomere und Salze.

4. Physiologisch verträgliche Salze der Verbindungen nach einem der Ansprüche 1 bis 3 mit anorganischen oder organischen Säuren.

5. Arzneimittel, enthaltend eine Verbindung nach einem der Ansprüche 1 bis 3 oder ein physiologisch verträgliches Salz gemäß Anspruch 4 neben gegebenenfalls einem oder mehreren inerten Trägerstoffen und/oder Verdünnungsmitteln.

6. Verwendung einer Verbindung nach mindestens einem der Ansprüche 1 bis 4 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

7. Verfahren zur Herstellung eines Arzneimittels gemäß Anspruch 5, **dadurch gekennzeichnet, daß** auf nichtchemischen Weg eine Verbindung nach mindestens einem der Ansprüche 1 bis 4 in einen oder mehrere inerte Trägerstoffe und/oder Verdünnungsmittel eingearbeitet wird.

8. Verfahren zur Herstellung der Verbindungen der allgemeinen Formel I gemäß den Ansprüchen 1 bis 4, **dadurch gekennzeichnet, daß**
a) eine Verbindung der allgemeinen Formel 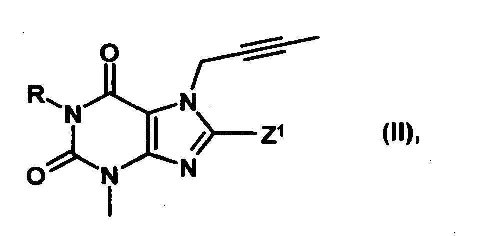 in der
R wie in Anspruch 1 erwähnt definiert ist und
Z¹ eine Austrittsgruppe wie ein Halogenatom, eine substituierte Hydroxy-, Mercapto-, Sulfinyl-, Sulfonyl- oder Sulfonyloxygruppe darstellt,
mit 3-Aminopiperidin, dessen Enantiomeren oder dessen Salzen umgesetzt wird, oder
b) eine Verbindung der allgemeinen Formel 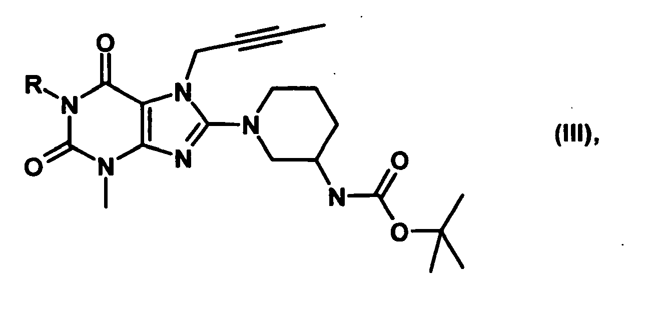 in der R wie in Anspruch 1 erwähnt definiert ist, entschützt wird,
und/oder
anschließend gegegebenenfalls während der Umsetzung verwendete Schutzgruppen abgespalten werden und/oder
die so erhaltenen Verbindungen der allgemeinen Formel I in ihre Enantiomeren und/oder Diastereomeren aufgetrennt werden und/oder
die erhaltenen Verbindungen der Formel I in ihre Salze, insbesondere für die pharmazeutische Anwendung in ihre physiologisch verträglichen Salze mit anorganischen oder organischen Säuren, übergeführt werden.

9. Eine Verbindung der allgemeinen Formel 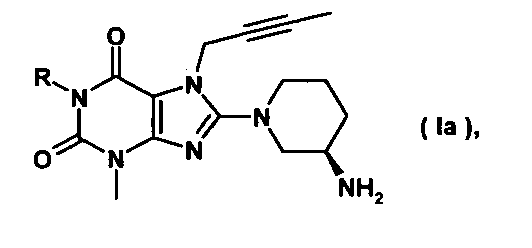 in der R eine (3-Cyanopyridin-2-yl)methyl-Gruppe bedeutet, oder deren physiologisch verträgliches Salz mit einer anorganischen oder organischen Säure.

10. Arzneimittel enthaltend eine Verbindung der allgemeinen Formel 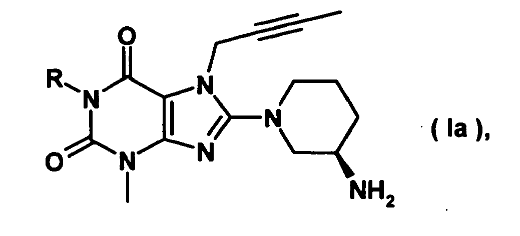 in der R eine (3-Cyanopyridin-2-yl)methyl-Gruppe bedeutet, oder deren physiologisch verträgliches Salz mit einer anorganischen oder organischen Säure, neben gegebenenfalls einem oder mehreren inerten Trägerstoffen und/oder Verdünnungsmitteln.

11. Verwendung einer Verbindung der allgemeinen Formel 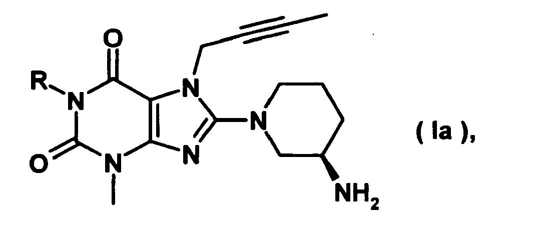 in der R eine (3-Cyanopyridin-2-yl)methyl-Gruppe bedeutet, oder deren physiologisch verträgliches Salz mit einer anorganischen oder organischen Säure, zur Herstellung eines Arzneimittels, das zur Behandlung von Diabetes mellitus Typ II oder Adipositas geeignet ist.

12. Verfahren zur Herstellung eines Arzneimittels gemäß Anspruch 10, **dadurch gekennzeichnet, daß** auf nichtchemischen Weg eine Verbindung der allgemeinen Formel 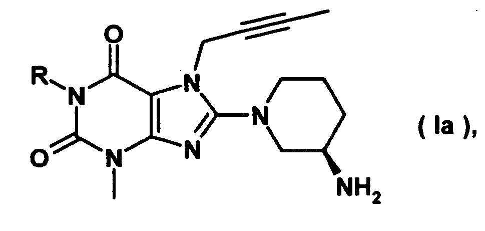 in der R eine (3-Cyanopyridin-2-yl)methyl-Gruppe bedeutet, oder deren physiologisch verträgliches Salz mit einer anorganischen oder organischen Säure, in einen oder mehrere inerte Trägerstoffe und/oder Verdünnungsmittel eingearbeitet wird.

13. Die Verbindung 1-[(3-Cyano-pyridin-2-yl)methyl]-3-methyl-7-(2-butin-1-yl)-8-((R)-3-amino-piperidin-1-yl)-xanthin in Form eines physiologisch verträglichen Salzes mit einer anorganischen oder organischen Säure.

14. Arzneimittel enthaltend 1-[(3-Cyano-pyridin-2-yl)methyl]-3-methyl-7-(2-butin-1-yl)-8-((*R*)-3-amino-piperidin-1-yl)-xanthin in Form eines physiologisch verträglichen Salzes mit einer anorganischen oder organischen Säure, neben gegebenenfalls einem oder mehreren inerten Trägerstoffen und/oder Verdünnungsmitteln.

15. Verwendung der Verbindung 1-[(3-Cyano-pyridin-2-yl)methyl]-3-methyl-7-(2-butin-1-yl)-8-((*R*)-3-amino-piperidin-1-yl)-xanthin in Form ihres physiologisch verträglichen Salzes mit einer anorganischen oder organischen Säure, zur Herstellung eines Arzneimittels, das zur Behandlung von Diabetes mellitus Typ II oder Adipositas geeignet ist.

16. Verfahren zur Herstellung eines Arzneimittels gemäß Anspruch 14, **dadurch gekennzeichnet, daß** auf nichtchemischen Weg 1-[(3-Cyano-pyridin-2-yl)methyl]-3-methyl-7-(2-butin-1-yl)-8-((*R*)-3-amino-piperidin-1-yl)-xanthin in Form eines physiologisch verträglichen Salzes mit einer anorganischen oder organischen Säure, in einen oder mehrere inerte Trägerstoffe und/oder Verdünnungsmittel eingearbeitet wird.

## Claims

1. Compounds of general formula 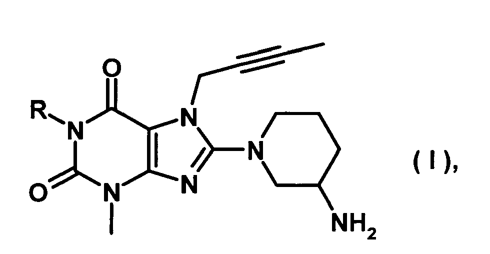 wherein
R denotes a benzyl, 2-fluorobenzyl, 3-fluorobenzyl, 4-fluorobenzyl, 2,6-difluoro-benzyl, 3,4-difluoro-benzyl, 2-chlorobenzyl, 3-chlorobenzyl or 4-chlorobenzyl group,
a 2-trifluoromethyl-benzyl, 3-trifluoromethyl-benzyl or 4-trifluoromethyl-benzyl group,
a 3-trifluoromethoxy-benzyl or 4-trifluoromethoxy-benzyl group,
a 2-cyanobenzyl, 3-cyanobenzyl or 4-cyanobenzyl group,
a 2,6-dicyanobenzyl, 3,4-dicyanobenzyl, 3,5-dicyanobenzyl, 2-trifluoromethyl-4-cyano-benzyl, 3-nitro-4-cyano-benzyl, 2-cyano-3-methoxy-benzyl, 2-cyano-4-methoxy-benzyl, 2-cyano-5-methoxy-benzyl, 2-cyano-4-fluoro-benzyl, 2-cyano-5-fluoro-benzyl, 2-cyano-6-fluoro-benzyl, 3-cyano-4-fluoro-benzyl, 4-cyano-3-fluoro-benzyl, 2-fluoro-4-cyano-benzyl, 2-cyano-3-chlorobenzyl, 2-chloro-4-cyano-benzyl or 2-cyano-4-bromobenzyl group,
a 2-methoxy-benzyl, 3-methoxy-benzyl, 4-methoxy-benzyl, 2-fluoro-3-methoxy-benzyl, 2-fluoro-4-methoxy-benzyl, 2-fluoro-5-methoxy-benzyl, 3-fluoro-4-methoxy-benzyl, 3,4-dimethoxy-benzyl, 3,5-dimethoxybenzyl or 3,4-dimethoxy-6-fluoro-benzyl group,
a (benzo[1,3]dioxol-5-yl)methyl group,
a [(4-cyano-benzo[1,3]dioxol-5-yl)methyl group,
a 2-(3-cyclopropyloxy-phenyl)-2-oxo-ethyl, 2-(3-cyclopropylmethoxy-phenyl)-2-oxo-ethyl or 2-(3-cyclobutyloxy-phenyl)-2-oxo-ethyl group,
a 2-oxo-2-[2-(pyridin-3-yl)-phenyl]-ethyl or 2-oxo-2-[2-(pyridin-4-yl)-phenyl]-ethyl group,
a (3-cyano-naphthalen-1-yl)methyl, (1,4-dicyano-naphthalen-2-yl)methyl or (2,4-dimethoxy-naphthalen-1-yl)methyl group,
a (furan-2-yl)methyl, (furan-3-yl)methyl, (5-bromo-furan-2-yl)methyl, (5-methyl-furan-2-yl)methyl, (5-cyano-furan-2-yl)methyl or (5-methoxycarbonyl-furan-2-yl)methyl group,
a (pyridin-2-yl)methyl, (6-fluoro-pyridin-2-yl)methyl or (5-methoxy-pyridin-2-yl)methyl group,
a (3-cyanopyridin-2-yl)methyl, (6-cyanopyridin-2-yl)methyl, (5-cyano-pyridin-2-yl)methyl, (4-cyano-pyridin-2-yl)methyl, (4-cyano-pyridin-3-yl)methyl, (3-cyano-pyridin-4-yl)methyl, (2-cyano-pyridin-3-yl)methyl, (2-cyano-pyridin-4-yl)methyl, (5-cyano-pyridin-3-yl)methyl, (6-cyano-pyridin-3-yl)methyl or (5-cyano-6-methoxy-pyridin-2-yl)methyl group,
a (6-phenyl-pyridin-2-yl)methyl or a ([2,2']bipyridinyl-6-yl)methyl group,
a (pyrimidin-2-yl)methyl, (4-methyl-pyrimidin-2-yl)methyl or (4,6-dimethyl-pyrimidin-2-yl)methyl group,
a (2-phenyl-pyrimidin-4-yl)methyl or (4-phenyl-pyrimidin-2-yl)methyl group,
a [(1-methyl-1 H-benzotriazol-5-yl)methyl] group,
a (6-fluoro-quinolin-2-yl)methyl, (7-fluoro-quinolin-2-yl)methyl, (2-methyl-quinolin-4-yl)methyl, (3-cyano-quinolin-2-yl)methyl, (3-cyano-4-methyl-quinolin-2-yl)methyl, (4-cyano-quinolin-2-yl)methyl, (5-cyano-quinolin-2-yl)methyl, (8-cyano-quinolin-2-yl)methyl, (6-amino-quinolin-2-yl)methyl, (8-amino-quinolin-2-yl)methyl, (4-methoxy-quinolin-2-yl)methyl, (6-methoxy-quinolin-2-yl)methyl, (6,7-dimethoxy-quinolin-2-yl)methyl or (8-cyano-quinolin-7-yl)methyl group,
a (1-cyano-isoquinolin-3-yl)methyl, (4-cyano-isoquinolin-1-yl)methyl, (4-cyano-isoquinolin-3-yl)methyl or [(4-(pyridin-2-yl)-isoquinolin-1-yl]methyl group,
a (quinazolin-6-yl)methyl, (quinazolin-7-yl)methyl, (2-methyl-quinazolin-4-yl)methyl, (4,5-dimethyl-quinazolin-2-yl)methyl, (4-ethyl-quinazolin-2-yl)methyl, (4-cyclopropyl-quinazolin-2-yl)methyl, (2-phenyl-quinazolin-4-yl)methyl, (4-cyano-quinazolin-2-yl)methyl, (4-phenylamino-quinazolin-2-yl)methyl or (4-benzylamino-quinazolin-2-yl)methyl group,
a (quinoxalin-5-yl)methyl, (quinoxalin-6-yl)methyl or (2,3-dimethyl-quinoxalin-6-yl)methyl group, or
a ([1,5]naphthyridin-3-yl)methyl group,
the tautomers, enantiomers, diastereomers, the mixtures and the salts thereof.

2. A compound of general formula 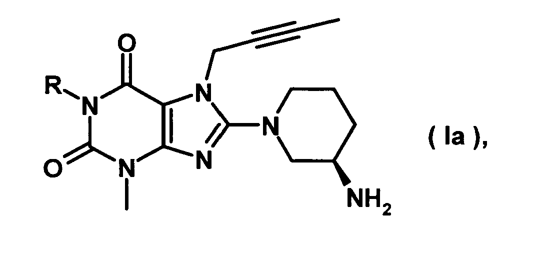 wherein R is defined as in claim 1, and the tautomers and salts thereof.

3. A compound of general formula 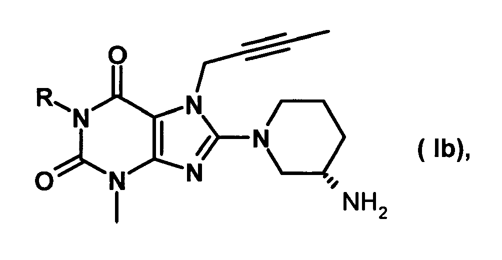 wherein R is defined as in claim 1, and the tautomers and salts thereof.

4. Physiologically acceptable salts of the compounds according to one of claims 1 to 3 with inorganic or organic acids.

5. Pharmaceutical compositions containing a compound according to at least one of claims 1 to 3 or a physiologically acceptable salt according to claim 4, optionally together with one or more inert carriers and/or diluents.

6. Use of a compound according to at least one of claims 1 to 4 for preparing a pharmaceutical composition which is suitable for treating type I and II diabetes mellitus, arthritis, obesity, allograft transplantation and calcitonin-induced osteoporosis.

7. Process for preparing a pharmaceutical composition according to claim 5, **characterised in that** a compound according to at least one of claims 1 to 4 is incorporated in one or more inert carriers and/or diluents by a non-chemical method.

8. Process for preparing the compounds of general formula I according to claims 1 to 4, **characterised in that**
a) a compound of general formula 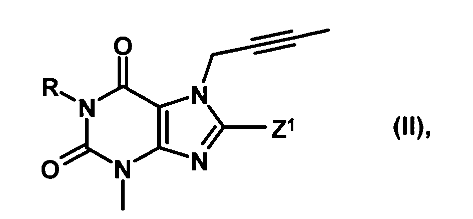 wherein
R is defined as in claim 1 and
Z¹ denotes a leaving group such as a halogen atom, a substituted hydroxy, mercapto, sulphinyl, sulphonyl or sulphonyloxy group,
is reacted with 3-aminopiperidine, the enantiomers or the salts thereof, or
b) a compound of general formula 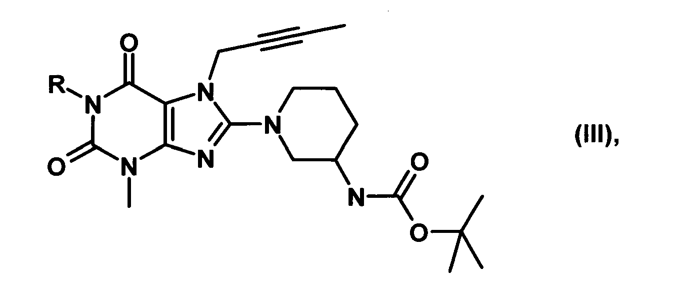
wherein R is defined as in claim 1, is deprotected,
and/or
any protecting groups used during the reaction are then cleaved and/or
the compounds of general formula I thus obtained are resolved into their enantiomers and/or diastereomers and/or
the compounds of formula I thus obtained are converted into their salts, particularly for pharmaceutical use into the physiologically acceptable salts thereof with inorganic or organic acids.

9. A compound of general formula 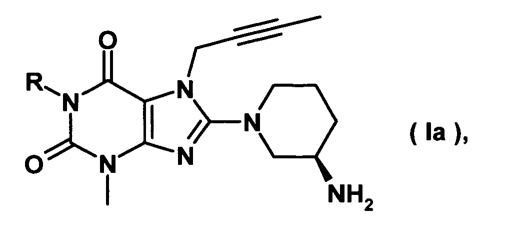 wherein R denotes a (3-cyanopyridin-2-yl)methyl group, or a physiologically acceptable salt thereof with an inorganic or organic acid.

10. Pharmaceutical composition containing a compound of general formula 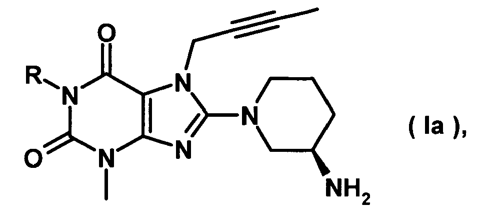 wherein R denotes a (3-cyanopyridin-2-yl)methyl group, or a physiologically acceptable salt thereof with an inorganic or organic acid, optionally together with one or more inert carriers and/or diluents.

11. Use of a compound of general formula 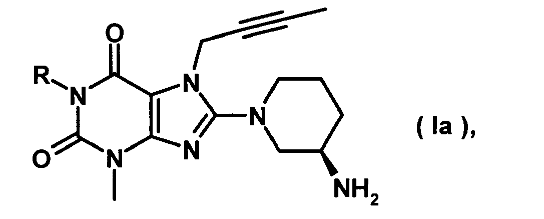 wherein R denotes a (3-cyanopyridin-2-yl)methyl group, or a physiologically acceptable salt thereof with an inorganic or organic acid, for preparing a pharmaceutical composition which is suitable for treating type II diabetes mellitus or obesity.

12. Process for preparing a pharmaceutical composition according to claim 10, **characterised in that** a compound of general formula 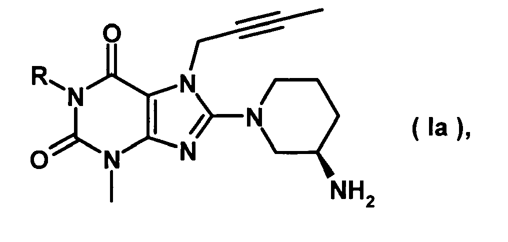 wherein R denotes a (3-cyanopyridin-2-yl)methyl group, or a physiologically acceptable salt thereof with an inorganic or organic acid, is incorporated in one or more inert carriers and/or diluents by a non-chemical method.

13. The compound 1-[(3-cyano-pyridin-2-yl)methyl]-3-methyl-7-(2-butyn-1-yl)-8-((R)-3-amino-piperidin-1-yl)-xanthine in a physiologically acceptable salt form with an inorganic or organic acid.

14. Pharmaceutical composition containing 1-[(3-cyano-pyridin-2-yl)methyl]-3-methyl-7-(2-butyn-1-yl)-8-((R)-3-amino-piperidin-1-yl)-xanthine in a physiologically acceptable salt form with an inorganic or organic acid, optionally together with one or more inert carriers and/or diluents.

15. Use of the compound 1-[(3-cyano-pyridin-2-yl)methyl]-3-methyl-7-(2-butyn-1-yl)-8-((R)-3-amino-piperidin-1-yl)-xanthine in a physiologically acceptable salt form with an inorganic or organic acid for preparing a pharmaceutical composition which is suitable for treating type II diabetes mellitus or obesity.

16. Process for preparing a pharmaceutical composition according to claim 14, **characterised in that** 1-[(3-cyano-pyridin-2-yl)methyl]-3-methyl-7-(2-butyn-1-yl)-8-((R)-3-amino-piperidin-1-yl)-xanthine in a physiologically acceptable salt form with an inorganic or organic acid is incorporated in one or more inert carriers and/or diluents by a non-chemical method.

## Revendications

1. Composés de la formule générale : 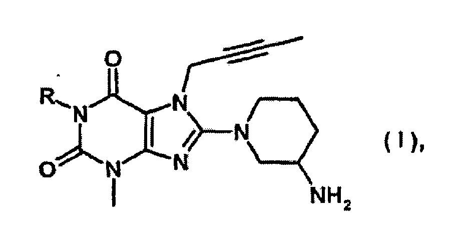 dans laquelle :
R représente un groupe benzyle, 2-fluorobenzyle, 3-fluorobenzyle, 4-fluorobenzyle, 2,6-difluorobenzyle, 3,4-difluorobenzyle, 2-chlorobenzyle, 3-chlorobenzyle ou 4-chlorobenzyle, un groupe 2-trifluorométhylbenzyle, 3-trifluorométhylbenzyle ou 4-trifluorométhylbenzyle,
un groupe 3-trifluorométhoxybenzyle ou 4-trifluorométhoxybenzyle,
un groupe 2-cyanobenzyle, 3-cyanobenzyle ou 4-cyanobenzyle,
un groupe 2,6-dicyanobenzyle, 3,4-dicyanobenzyle, 3,5-dicyanobenzyle, 2-trifluorométhyl-4-cyanobenzyle, 3-nitro-4-cyanobenzyle, 2-cyano-3-méthoxybenzyle, 2-cyano-4-méthoxybenzyle, 2-cyano-5-méthoxybenzyle, 2-cyano-4-fluorobenzyle, 2-cyano-5-fluorobenzyle, 2-cyano-6-fluorobenzyle, 3-cyano-4-fluorobenzyle, 4-cyano-3-fluorobenzyle, 2-fluoro-4-cyanobenzyle, 2-cyano-3-chlorobenzyle, 2-chloro-4-cyanobenzyle ou 2-cyano-4-bromobenzyle,
un groupe 2-méthoxybenzyle, 3-méthoxybenzyle, 4-méthoxybenzyle, 2-fluoro-3-méthoxybenzyle, 2-fluoro-4-méthoxybenzyle, 2-fluoro-5-méthoxybenzyle, 3-fluoro-4-méthoxybenzyle, 3,4-diméthoxybenzyle, 3,5-diméthoxybenzyle ou 3,4-diméthoxy-6-fluorobenzyle,
un groupe (benzo[1,3]dioxo-5-yl)méthyle,
un groupe (4-cyanobenzo[1,3]dioxol-5-yl)méthyle,
un groupe 2-(3-cyclopropyloxyphényl)-2-oxoéthyle, 2-(3-cyclopropylméthoxyphényl)-2-oxoéthyle ou 2-(3-cyclobutyloxyphényl)-2-oxoéthyle,
un groupe 2-oxo-2-[2-(pyridin-3-yl)phényl]éthyle ou 2-oxo-2-[2-(pyridin-4-yl)phényl]éthyle,
un groupe (3-cyanonaphtalèn-1-yl)méthyle, 1,4-dicyanonaphtalèn-2-yl)méthyle ou (2,4-diméthoxynaphtalèn-1-yl)méthyle,
un groupe (furan-2-yl)méthyle, (furan-3-yl)méthyle, (5-bromofuran-2-yl)méthyle, (5-méthylfuran-2-yl)méthyle, (5-cyanofuran-2-yl)méthyle ou (5-méthoxycarbonylfuran-2-yl)méthyle,
un groupe (pyridin-2-yl)méthyle, (6-fluoropyridin-2-yl)méthyle ou (5-méthoxy-pyridin-2-yl)méthyle,
un groupe (3-cyanopyridin-2-yl)méthyle, (6-cyanopyridin-2-yl)méthyle, (5-cyano-pyridin-2-yl)méthyle, (4-cyanopyridin-2-yl)méthyle, (4-cyanopyridin-3-yl)méthyle, (3-cyanopyridin-4-yl)méthyle, (2-cyanopyridin-3-yl)méthyle, (2-cyanopyridin-4-yl)méthyle, (5-cyanopyridin-3-yl)méthyle, (6-cyanopyridin-3-yl)méthyle ou (5-cyano-6-méthoxypyridin-2-yl)méthyle,
un groupe (6-phénylpyridin-2-yl)méthyle ou ([2,2']-bipyridinyl-6-yl)méthyle, un groupe (pyrimidin-2-yl)méthyle, (4-méthylpyrimidin-2-yl)méthyle ou (4,6-diméthylpyrimidin-2-yl)méthyle,
un groupe (2-phénylpyrimidin-4-yl)méthyle ou (4-phénylpyrimidin-2-yl)méthyle,
un groupe [(1-méthyl-1H-benzotriazol-5-yl)méthyle],
un groupe (6-fluoroquinoléin-2-yl)méthyle, (7-fluoroquinoléin-2-yl)méthyle, (2-méthylquinoléin-4-yl)méthyle, (3-cyanoquinoléin-2-yl)méthyle, (3-cyano-4-méthylquinoléin-2-yl)méthyle, (4-cyanoquinoléin-2-yl)méthyle, (5-cyanoquinoléin-2-yl)méthyle, (8-cyanoquinoléin-2-yl)méthyle, (6-aminoquinoléin-2-yl)méthyle, (8-aminoquinoléin-2-yl)méthyle, (4-méthoxyquinoléin-2-yl)méthyle, (6-méthoxy-quinoléin-2-yl)méthyle, (6,7-diméthoxyquinoléin-2-yl)méthyle ou (8-cyano-quinoléin-7-yl)méthyle,
un groupe (1-cyanoisoquinoléin-3-yl)méthyle, (4-cyanoisoquinoléin-1-yl)méthyle, (4-cyanoisoquinoléin-3-yl)méthyle ou [(4-pyridin-2-yl)isoquinoléin-1-yl]méthyle, un groupe (quinazolin-6-yl)méthyle, (quinazolin-7-yl)méthyle, (2-méthylquinazolin-4-yl)méthyle, (4,5-diméthylquinazolin-2-yl)méthyle, (4-éthylquinazolin-2-yl)méthyle, (4-cyclopropylquinazolin-2-yl)méthyle, (2-phénylquinazolin-4-yl)méthyle, (4-cyanoquinazolin-2-yl)méthyle, (4-phénylaminoquinazolin-2-yl)méthyle ou (4-benzylaminoquinazolin-2-yl)méthyle,
un groupe (quinoxalin-5-yl)méthyle, (quinoxalin-6-yl)méthyle ou (2,3-diméthylquinoxalin-6-yl)méthyle,
un groupe ([1,5]naphtyridin-3-yl)méthyle,
leurs tautomères, énantiomères, diastéréoisomères, leurs mélanges et leurs sels.

2. Composé de la formule générale : 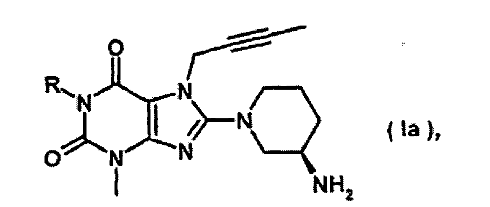 dans laquelle R est défini comme indiqué à la revendication 1, ainsi que leurs tautomères et sels.

3. Composé de la formule générale : 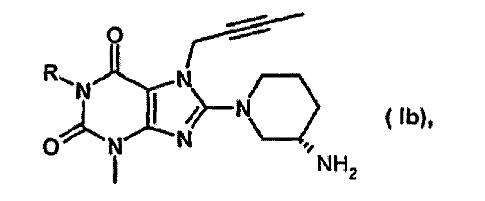 dans laquelle R est défini comme indiqué à la revendication 1, ainsi que leurs tautomères et sels.

4. Sels physiologiquement acceptables des composés selon l'une des revendications 1 à 3, avec des acides inorganiques ou organiques.

5. Produit pharmaceutique contenant un composé selon l'une des revendications 1 à 3 ou un sel physiologiquement acceptable selon la revendication 4, avec le cas échéant, un ou plusieurs supports et/ou agents de dilution inertes.

6. Utilisation d'un composé selon au moins une des revendications 1 à 4, pour la préparation d'un produit pharmaceutique, qui est approprié pour le traitement du diabète de type I et de type II, l'arthrite, l'adiposité, la transplantation d'allogreffe et l'ostéoporose engendrée par la calcitonine.

7. Procédé de préparation d'un produit pharmaceutique selon la revendication 5, **caractérisé en ce que** l'on incorpore par une voie non chimique, un composé selon au moins l'une des revendications 1 à 4, dans un ou plusieurs supports et/ou agents de dilution inertes.

8. Procédé de préparation des composés de la formule générale I selon les revendications 1 à 4, **caractérisé en ce que**
a) l'on fait réagir un composé de formule générale : 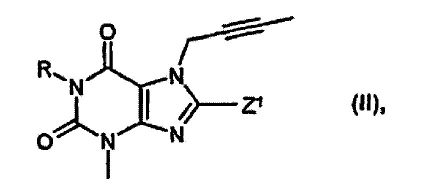 dans laquelle
R est défini comme indiqué à la revendication 1, et
Z¹ est un groupe partant, tels qu'un atome d'halogène, un groupe hydroxy, mercapto, sulfinyle, sulfonyle ou sulfonyloxy substitué,
avec de la 3-aminopipéridine, ses énantiomères ou ses sels,
ou
b) l'on procède à la déprotection d'un composé de la formule générale : 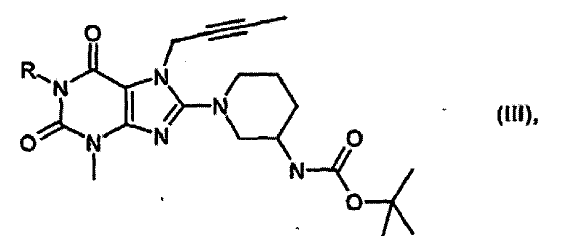
dans laquelle
R est défini comme indiqué à la revendication 1, est déprotégé,
et/ou
ensuite, le cas échéant pendant la transformation, les groupes protecteurs utilisés sont
clivés, et/ou
les composés ainsi obtenus de la formule générale I sont séparés en leurs énantiomères et/ou diastéréoisomères, et/ou
les composés obtenus de formule I sont convertis en leurs sels, en particulier pour l'utilisation pharmaceutique, en leur sels physiologiquement compatibles avec des acides inorganiques ou organiques.

9. Composé de la formule générale : 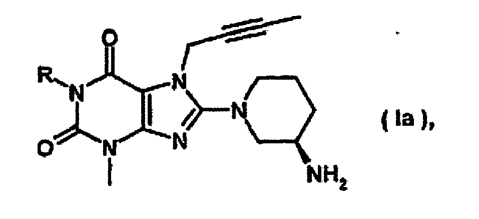 dans laquelle :
R représente un groupe (3-cyanopyridin-2-yl)méthyle, ou l'un de ses sels physiologiquement compatibles avec des acides inorganiques ou organiques.

10. Produit pharmaceutique contenant un composé de la formule générale : 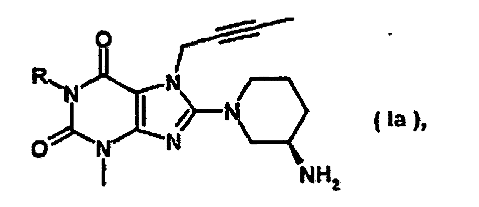 dans laquelle :
R représente un groupe (3-cyanopyridin-2-yl)méthyle, ou l'un de ses sels physiologiquement compatibles avec des acides inorganiques ou organiques, avec le cas échéant, un ou plusieurs supports et/ou agents de dilution inertes.

11. Utilisation d'un composé de la formule générale : 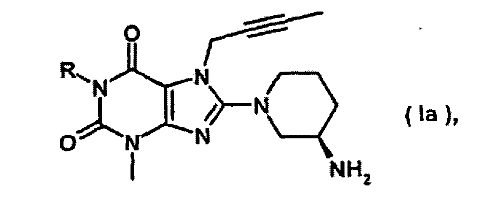 dans laquelle :
R représente un groupe (3-cyanopyridin-2-yl)méthyle, ou l'un de ses sels physiologiquement compatibles avec des acides inorganiques ou organiques, pour la préparation d'un produit pharmaceutique, qui est approprié pour le traitement du diabète de type II ou l'adiposité.

12. Procédé de préparation d'un produit pharmaceutique selon la revendication 10, **caractérisé en ce que** l'on incorpore par une voie non chimique, un composé de la formule générale : 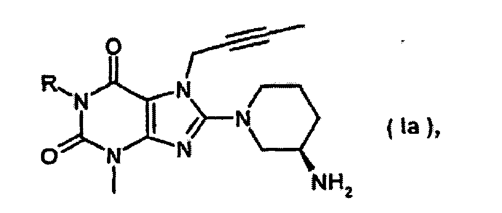 dans laquelle :
R représente un groupe (3-cyanopyridin-2-yl)méthyle, ou l'un de ses sels physiologiquement compatibles avec des acides inorganiques ou organiques, dans un
ou plusieurs supports et/ou agents de dilution inertes.

13. Le composé 1-[(3-cyanopyridin-2-yl)méthyl]-3-méthyl-7-(2-butyn-1-yl)-8-((R)-3-aminopipéridin-1-yl)xanthine sous la forme d'un sel physiologiquement compatible avec un acide inorganique ou organique.

14. Produit pharmaceutique contenant 1-[(3-cyanopyridin-2-yl)méthyl]-3-méthyl-7-(2-butyn-1-yl)-8-((R)-3-aminopipéridin-1-yl)xanthine sous la forme d'un sel physiologiquement compatible avec un acide inorganique ou organique, avec le cas échéant, un ou plusieurs supports et/ou agents de dilution inertes.

15. Utilisation du composé 1-[(3-cyanopyridin-2-yl)méthyl]-3-méthyl-7-(2-butyn-1-yl)-8-((R)-3-aminopipéridin-1-yl)xanthine sous la forme d'un sel physiologiquement compatible avec un acide inorganique ou organique, pour la préparation d'un produit pharmaceutique, qui est approprié pour le traitement du diabète de type II ou l'adiposité.

16. Procédé de préparation d'un produit pharmaceutique selon la revendication 14, **caractérisé en ce que** l'on incorpore par une voie non chimique, 1-[(3-cyanopyridin-2-yl)méthyl]-3-méthyl-7-(2-butyn-1-yl)-8-((R)-3-aminopipéridin-1-yl)xanthine sous la forme d'un sel physiologiquement compatible avec un acide inorganique ou organique, dans un ou plusieurs supports et/ou agents de dilution inertes.
